(19) Europäisches Patentamt
European Patent Office
Office européen des brevets

(11) EP 3 690 438 A1

(12) **EUROPEAN PATENT APPLICATION**
published in accordance with Art. 153(4) EPC

(43) Date of publication:
**05.08.2020 Bulletin 2020/32**

(21) Application number: **18861409.3**

(22) Date of filing: **27.09.2018**

(51) Int Cl.:
***G01N 33/49*** *(2006.01)*

(86) International application number:
**PCT/JP2018/035978**

(87) International publication number:
**WO 2019/065854 (04.04.2019 Gazette 2019/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.09.2017 JP 2017186273**

(71) Applicant: **Renatech Co., Ltd.**
**Isehara-shi, Kanagawa 259-1114 (JP)**

(72) Inventors:
- **INAGAKI, Seiichi**
  **Isehara-shi, Kanagawa 259-1114 (JP)**
- **OKAMOTO, Naoyuki**
  **Isehara-shi, Kanagawa 259-1114 (JP)**
- **MIKAMI, Haruo**
  **Chiba-shi, Chiba 260-8717 (JP)**
- **MIYAGI, Yohei**
  **Yokohama-shi, Kanagawa 241-8515 (JP)**

(74) Representative: **Prüfer & Partner mbB**
**Patentanwälte · Rechtsanwälte**
**Sohnckestraße 12**
**81479 München (DE)**

# (54) CANCER RISK EVALUATION METHOD AND CANCER RISK EVALUATION SYSTEM

(57) A cancer risk evaluation method is provided, which makes it possible to estimate the risk of suffering from cancer of a subject with high accuracy, which do not have the disadvantages of early degeneration and high cost that arise in the case where the in-blood amino acid concentrations are utilized, and which are capable of estimating which site of cancer a subject has.

This method includes the step S1 of measuring the concentrations of a set of evaluation elements contained in a serum sample 2 taken from a subject, the step S2 of applying concentration data of the set of elements thus measured and age data of the subject to a discriminant function or functions for discriminating to which of a case group and a control group the subject belongs to perform an operation; and the step S3 of obtaining an indicator for discriminating whether or not the subject suffers from any type of cancer based on a correlation among the set of evaluation elements obtained in the step S2. As the set of evaluation elements, a combination of 17 elements of Na, Mg, P, S, K, Ca, Fe, Cu, Zn, Se, Rb, Sr, As, Mo, Cs, Co, and Ag is used.

FIG. 1

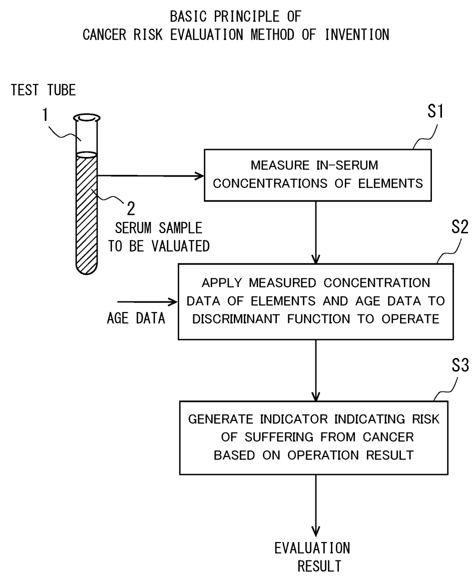


EP 3 690 438 A1

## Description

TECHNICAL FIELD

The present invention relates to a cancer risk evaluation method and a cancer risk evaluation system and more particularly, to a cancer risk evaluation method and a cancer risk evaluation system that use an indicator which is obtained by utilizing the concentration balance of elements (correlations among the concentrations of a set of evaluation elements) contained in a human serum.

BACKGROUND ART

As the diagnostic method of cancer, the method of direct observation or touching (e.g., palpation, endoscopic examination, etc.), the method of judging with images that reflect the inside of a human body (e.g., roentgenographic examination, CT examination, MRI examination, PET examination, etc.), and the method of examining blood or cells (e.g., blood test, cytodiagnosis, biopsy, etc.) are known.

However, the method of direct observation or touching has a disadvantage that the examination target (affected part) is restricted to breast, rectum, stomach, large intestine, and so on. The method of judging with images has a disadvantage that not only that the detection sensitivity is low but also that the subject is exposed to radiation, although this method is readily carried out. On the other hand, the method of examining blood or cells is preferred because the burden on the patient is light and the detection sensitivity is high. In particular, if diagnosis is made possible by analyzing blood which is taken from a patient, it is more preferred; this is because the burden on the patient is reduced to a low level and at the same time, diagnosis can be carried out even in the group or mass examination.

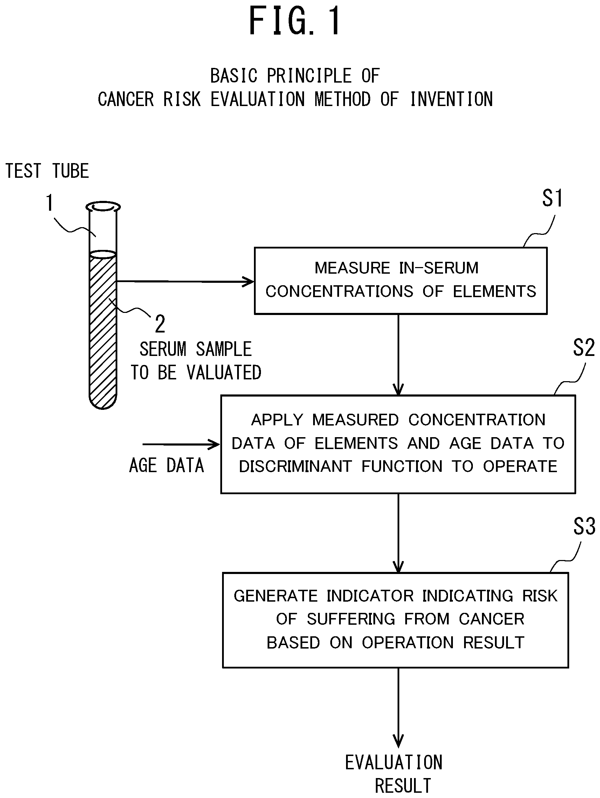

Conventionally, it is known that the concentrations of amino acids contained in the blood which is taken from a patient vary in association with the onset of cancer. Patent Literature 1 discloses a method of diagnosing lung cancer by measuring the concentrations of in-blood amino acids of a patient utilizing such the relationship as described here. This method is an evaluation method of lung cancer characterized in that the step of obtaining amino acid concentration data about the values of the amino acid concentrations in the blood which is picked up from an evaluation subject, and the step of evaluating the concentration reference for evaluating the state of lung cancer of the evaluation subject based on the concentration values of Lys and His contained in the amino acid concentration data of the evaluation subject which is obtained in the evaluation step are carried out. In addition, the step of evaluating the concentration reference may include the step of discriminating whether or not lung cancer develops with respect to the evaluation subject based on the concentration values of Lys and His contained in the amino acid concentration data of the evaluation subject which is obtained in the obtaining step. With this diagnosing method, it is described that the state of lung cancer can be accurately evaluated utilizing the amino acid concentrations which are relevant to the state of lung cancer within the in-blood amino acid concentrations. (See Claims 1 and 2, Paragraph 0106, and Figs. 1 to 3.)

On the other hand, it is known that the concentrations of trace elements contained in the blood have a relationship with the onset of cancer. For example, Non-Patent Literature 1 reports that the concentrations of copper (Cu) and zinc (Zn) and the concentration ratio of Cu/Zn in the serum of a breast cancer patient have a correlation with the development degree of condition of the patient. Moreover, Non-Patent Literature 2 reports that the concentration levels of cadmium (Cd) and lead (Pb) in the serum of a cancer patient are higher than those of a healthy person, and that the concentration levels of zinc (Zn), iron (Fe), and manganese (Mn) in the serum of a cancer patient are lower than those of a healthy person.

With the diagnosing method of the aforementioned Patent Literature 1, however, the amino acids in the blood degenerate early and thus, there is a disadvantage that the amino acid concentrations need to be quickly measured after collecting the blood. Moreover, since the diagnosis cost is high, there is another disadvantage that the diagnosis service becomes expensive. On the other hand, the method of diagnosing cancer utilizing the trace element concentrations in the serum like aforementioned Non-Patent Literatures 1 and 2 does not have the disadvantages of the diagnosing method of Patent Literature 1 and therefore, the cancer diagnosing method utilizing the in-serum trace element concentrations is preferred.

Taking this point into consideration, the applicant developed a novel cancer evaluation method and a novel cancer evaluation system and then, filed a patent application about them. The cancer evaluation method and the cancer evaluation system thus filed were already granted (see Patent Literature 2).

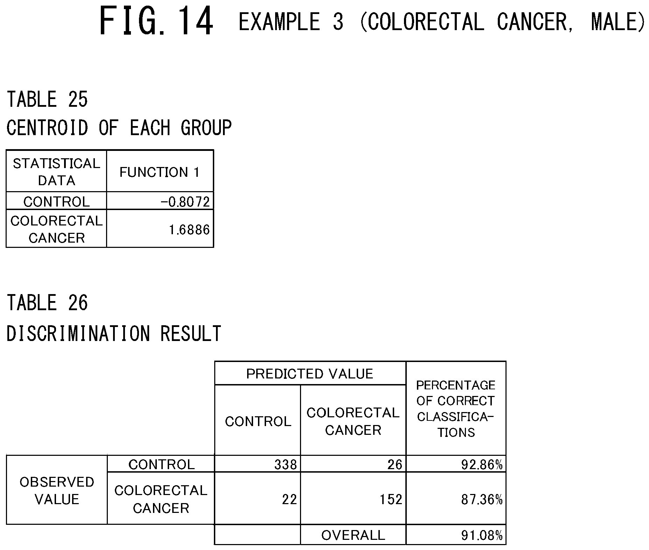

Patent Literature 2 discloses a cancer evaluation method that utilizes the correlations between the onset of cancer and the concentrations of elements contained in a human serum. This method, which was developed by one of the applicants of the present application, comprises the correlation operating step of operating a correlation among concentrations of a set of evaluation elements contained in a serum which is taken from a subject by applying concentration data of the set of evaluation elements to a discriminant function for discriminating which of a case group and a control group the subject belongs to; and the indicator obtaining step of obtaining an indicator for indicating whether or not the subject suffers from any type of cancer based on the correlation operated in the correlation operating step. In this method, as the set of evaluation elements, a combination of 7 elements of S, P, Mg, Zn, Cu, Ti, and Rb or a combination of 16 elements of Na, Mg, Al, P, K, Ca, Ti, Mn, Fe, Zn, Cu, Se, Rb, Ag, Sn, and S is chosen. This method have advantageous effects that the risk of suffering from cancer of a subject can be estimated with high accuracy, that the disadvantages of early degeneration and high cost that arise in the case where in-blood amino acid concentrations are utilized do not occur, and that this method can be applied easily to group or mass examinations (See Claims 1 and 2, Paragraphs 0036, 0057-0061, 0070-0074, and Figs. 1 and 14).

PRIOR ART LITERATURE

PATENT LITERATURE

Patent Literature 1: Japanese Examined Patent Publication No. 5,470,848
Patent Literature 2: Japanese Examined Patent Publication No. 6,082,478

NON-PATENT LITERATURE

Non-Patent Literature 1: Gupta SK et al., Serum trace elements and Cu/Zn ratio in breast cancer patients, Journal of Surgical Oncology, Mar. 46(3), 178-181, 1991
Non-Patent Literature 2: Necip Pirincci et al., Levels of Serum Trace Elements in Renal Cell Carcinoma Cases, Asian Pacific Journal of Cancer Prevention, Vol. 14(1), 499-502, 2013

SUMMARY OF THE INVENTION

PROBLEMS TO BE RESOLVED BY THE INVENTION

Regarding pancreatic cancer and endometrial cancer, there have not been suitable materials and/or indicators for screening and therefore, these cancers have not been considered as the diseases for which early detection and early treatment are effective. It is often that these cancers have already become advanced cancers when detected and at the same time, the prognosis of these cancers is bad. Accordingly, these cancers are termed intractable cancers and there is a strong demand for developing suitable screening methods for detecting these cancers.
Moreover, in addition to pancreatic cancer and endometrial cancer, the number of male patients having prostate cancer and colorectal cancer and that of female patients having breast cancer and colorectal cancer have been increasing. For this reason, it is necessary to develop suitable screening methods for detecting these cancers also.
Accordingly, the inventors found the possibility that makes it possible to develop a novel screening method for detecting a group of cancers, such as prostate cancer and colorectal cancer for male patients and breast cancer and colorectal cancer for female patients in addition to pancreatic cancer and endometrial cancer which are termed the intractable cancers, using a method of discriminating between cancer patients (a case group) and controls (a control group) that utilizes the concentration balance of trace elements contained in a serum which is disclosed in Patent Literature 2; thereafter, the inventors created the present invention.
An object of the present invention is to provide a cancer risk evaluation method and a cancer risk evaluation system that make it possible to estimate the risk of suffering from cancer of a subject with high accuracy, that do not have the disadvantages of early degeneration and high cost that arise in the case where the in-blood amino acid concentrations are utilized, and that are capable of estimating which site of cancer a subject has.
Another object of the present invention is to provide a cancer risk evaluation method and a cancer risk evaluation system that can be easily applied to group or mass examinations.
The other objects not specifically mentioned will become clear to those skilled in the art from the following description and drawings attached.

MEANS FOR SOLVING THE PROBLEMS

(1) According to a first aspect of the present invention, a cancer risk evaluation method is provided, which comprises:

the correlation operating step of operating a correlation among concentrations of a set of evaluation elements contained in a serum which is taken from a subject by applying concentration data of the set of evaluation elements and age data of the subject to a discriminant function or functions for discriminating which of a case group and a control group the subject belongs to; and

the indicator obtaining step of obtaining an indicator for discriminating whether or not the subject suffers from any type of cancer based on the correlation operated in the correlation operating step;

wherein in the correlation operating step, a combination of 17 elements of Na, Mg, P, S, K, Ca, Fe, Cu, Zn, Se, Rb, Sr, As, Mo, Cs, Co, and Ag is used as the set of evaluation elements; and

in the indicator obtaining step, the indicator is generated based on a discriminant score or scores calculated by applying the concentration data and the age data to the discriminant function or functions which is/are used in the correlation operating step.

With the cancer risk evaluation method according to the first aspect of the present invention, in the correlation operating step, the concentration data of the set of evaluation elements contained in the serum which is taken from the subject and the age data of the subject are applied to the discriminant function or functions for discriminating which of the case group and the control group the subject belongs to, thereby operating the correlation among the concentrations of the set of evaluation elements in the serum. The combination of aforementioned 17 elements is used as the set of evaluation elements.

Moreover, in the indicator obtaining step, the indicator for discriminating whether or not the subject suffers from any type of cancer is obtained based on the correlation which is obtained in the correlation operating step. The indicator is generated based on the discriminant score or scores which is/are obtained by applying the concentration data and the age data to the discriminant function or functions which is/are used in the correlation operating step.

Accordingly, the risk of suffering from cancer of the subject can be estimated with high accuracy and at the same time, the disadvantages of early degeneration and high cost that arise in the case where the in-blood amino acid concentrations are utilized do not occur.

Furthermore, it is known which of the concentration data of the aforementioned 17 elements as the set of evaluation elements is/are significant for discrimination in the correlation operating step, and the one or more elements which is/are judged significant for discrimination is/are changed according to the type of cancer. As a result, which site of cancer the subject has can be estimated also.

Furthermore, which of the case group and the control group the subject belongs to can be discriminated by automatic operation with a computer using the concentration data of the set of evaluation elements in the serum which is taken from the subject and the age data of the subject. Accordingly, the discrimination can be performed easily and quickly even if the number of the subjects is large. This means that the method according to the first aspect of the present invention is easily applicable to group or mass examinations.

(2) In a preferred embodiment of the cancer risk evaluation method according to the first aspect of the present invention, in a case where the age data and the concentration data of the 9 elements of Na, P, S, Ca, Fe, Cu, Zn, Se, and Rb which are selected from the 17 elements used as the set of evaluation elements are judged significant for discrimination based on the correlation which is operated in the correlation operating step, and the subject is male, an estimate that a type of cancer of the subject is pancreatic cancer is included into the indicator. In this embodiment, there is an additional advantage that the risk of suffering from cancer can be notified to the subject while designating the type of cancer as "pancreatic cancer".

(3) In another preferred embodiment of the cancer risk evaluation method according to the first aspect of the present invention, in a case where the age data and the concentration data of the 10 elements of Na, P, S, K, Ca, Fe, As, Sr, Rb, and Mo which are selected from the 17 elements used as the set of evaluation elements are judged significant for discrimination based on the correlation which is operated in the correlation operating step, and the subject is male, an estimate that a type of cancer of the subject is prostate cancer is included into the indicator. In this embodiment, there is an additional advantage that the risk of suffering from cancer can be notified to the subject while designating the type of cancer as "prostate cancer".

(4) In still another preferred embodiment of the cancer risk evaluation method according to the first aspect of the present invention, in a case where the age data and the concentration data of the 10 elements of Na, P, S, K, Cu, Zn, Rb, Se, Mo, and Co which are selected from the 17 elements used as the set of evaluation elements are judged significant for discrimination based on the correlation which is operated in the correlation operating step, and the subject is male, an estimate that a type of cancer of the subject is colorectal cancer is included into the indicator. In this embodiment, there is an additional advantage that the risk of suffering from cancer can be notified to the subject while designating the type of cancer as "colorectal cancer".

(5) In a further preferred embodiment of the cancer risk evaluation method according to the first aspect of the present invention, in a case where the age data and the concentration data of the 6 elements of Mg, S, K, Ca, Fe, and Mo which are selected from the 17 elements used as the set of evaluation elements are judged significant for discrimination based on the correlation which is operated in the correlation operating step, and the subject is female, an estimate that a type of cancer of the subject is endometrial cancer is included into the indicator. In this embodiment, there is an additional advantage that the risk of suffering from cancer can be notified to the subject while designating the type of cancer as "endometrial cancer".

(6) In a further preferred embodiment of the cancer risk evaluation method according to the first aspect of the present invention, in a case where the age data and the concentration data of the 6 elements of Mg, P, S, Fe, Zn, and Cs which are selected from the 17 elements used as the set of evaluation elements are judged significant for discrimination based on the correlation which is operated in the correlation operating step, and the subject is female, an estimate that a type of cancer of the subject is breast cancer is included into the indicator. In this embodiment, there is an additional advantage that the risk of suffering from cancer can be notified to the subject while designating the type of cancer as "breast cancer".

(7) In a further preferred embodiment of the cancer risk evaluation method according to the first aspect of the present invention, in a case where the age data and the concentration data of the 10 elements of Na, P, S, Ca, Fe, Cu, Zn, As, Cs, and Ag which are selected from the 17 elements used as the set of evaluation elements are judged significant for discrimination based on the correlation which is operated in the correlation operating step, and the subject is female, an estimate that a type of cancer of the subject is colorectal cancer is included into the indicator. In this embodiment, there is an additional advantage that the risk of suffering from cancer can be notified to the subject while designating the type of cancer as "colorectal cancer".

(8) According to a second aspect of the present invention, a cancer risk evaluation system is provided, which comprises:

a data storage section for storing concentration data of a set of evaluation elements contained in a blood which is taken from a subject and age data of the subject;

a discriminant function generation section for generating a discriminant function or functions for discriminating which of a case group and a control group the subject belongs to; and

an evaluation result operation section for operating a correlation among concentrations of the set of evaluation elements contained in the serum by applying the concentration data of the subject and the age data thereof stored in the data storage section to a discriminant function or functions generated by the discriminant function generation section, thereby outputting an evaluation result that discriminates whether or not the subject suffers from any type of cancer based on the correlation;

wherein a combination of 17 elements of Na, Mg, P, S, K, Ca, Fe, Cu, Zn, Se, Rb, Sr, As, Mo, Cs, Co, and Ag is used as the set of evaluation elements; and

in the evaluation result operation section, a discriminant score or scores is/are calculated by applying the concentration data and the age data which are stored in the data storage section to the discriminant function or functions which is/are generated by the discriminant function generation section, and the evaluation result is generated based on the discriminant score or scores.

With the cancer risk evaluation system according to the second aspect of the resent invention, after concentration data of a set of evaluation elements contained in a serum which is taken from a subject and age data of the subject are stored in the data storage section, the evaluation result operation section applies the concentration data and the age data of the subject which are stored in the data storage section to a discriminant function or functions which is/are generated by the discriminant function generation section, thereby operating a correlation among concentrations of the set of evaluation elements in the serum. The combination of aforementioned 17 elements is used as the set of evaluation elements.

Moreover, the evaluation result operation section outputs an evaluation result that discriminates whether or not the subject suffers from any type of cancer based on the correlation obtained by the operations. The evaluation result is generated based on the discriminant score or scores which is/are obtained by applying the concentration data and the age data to the discriminant function or functions which is/are generated by the discriminant function generation section.

Accordingly, the risk of suffering from cancer of the subject can be estimated with high accuracy and at the same time, the disadvantages of early degeneration and high cost that arise in the case where the in-blood amino acid concentrations are utilized do not occur.

Furthermore, it is known which of the concentration data of the aforementioned 17 elements as the set of evaluation elements is/are significant for discrimination in the evaluation result operation section, and the one or more elements which is/are judged significant for discrimination is/are changed according to the type of cancer. As a result, which site of cancer the subject has can be estimated also.

Furthermore, which of the case group and the control group the subject belongs to can be discriminated by automatic operation with a computer using the concentration data of the set of evaluation elements in the serum which is taken from the subject and the age data of the subject. Accordingly, the discrimination can be performed easily and quickly even if the number of the subjects is large. This means that the cancer evaluation system according to the second aspect of the present invention is easily applicable to group or mass examinations.

(9) In a preferred embodiment of the cancer risk evaluation system according to the second aspect of the present invention, in a case where the age data and the concentration data of the 9 elements of Na, P, S, Ca, Fe, Cu, Zn, Se, and Rb which are selected from the 17 elements used as the set of evaluation elements are judged significant for discrimination based on the correlation which is operated by the evaluation result operation section, and the subject is male, an estimate that a type of cancer of the subject is pancreatic cancer is included into the evaluation result. In this embodiment, there is an additional advantage that the risk of suffering from cancer can be notified to the subject while designating the type of cancer as "pancreatic cancer".

(10) In another preferred embodiment of the cancer risk evaluation system according to the second aspect of the present invention, in a case where the age data and the concentration data of the 10 elements of Na, P, S, K, Ca, Fe, As, Sr, Rb, and Mo which are selected from the 17 elements used as the set of e valuation elements are judged significant for discrimination based on the correlation which is operated by the evaluation result operation section, and the subject is male, an estimate that a type of cancer of the subject is prostate cancer is included into the evaluation result. In this embodiment, there is an additional advantage that the risk of suffering from cancer can be notified to the subject while designating the type of cancer as "prostate cancer".

(11) In still another preferred embodiment of the cancer risk evaluation system according to the second aspect of the present invention, in a case where the age data and the concentration data of the 10 elements of Na, P, S, K, Cu, Zn, Rb, Se, Mo, and Co which are selected from the 17 elements used as the set of evaluation elements are judged significant for discrimination based on the correlation which is operated by the evaluation result operation section, and the subject is male, an estimate that a type of cancer of the subject is colorectal cancer is included into the evaluation result. In this embodiment, there is an additional advantage that the risk of suffering from cancer can be notified to the subject while designating the type of cancer as "colorectal cancer".

(12) In a further preferred embodiment of the cancer risk evaluation system according to the second aspect of the present invention, in a case where the age data and the concentration data of the 6 elements of Mg, S, K, Ca, Fe, and Mo which are selected from the 17 elements used as the set of evaluation elements are judged significant for discrimination based on the correlation which is operated by the evaluation result operation section, and the subject is female, an estimate that a type of cancer of the subject is endometrial cancer is included into the evaluation result. In this embodiment, there is an additional advantage that the risk of suffering from cancer can be notified to the subject while designating the type of cancer as "endometrial cancer".

(13) In a further preferred embodiment of the cancer risk evaluation system according to the second aspect of the present invention, in a case where the age data and the concentration data of the 6 elements of Mg, P, S, Fe, Zn, and Cs which are selected from the 17 elements used as the set of evaluation elements are judged significant for discrimination based on the correlation which is operated by the evaluation result operation section, and the subject is female, an estimate that a type of cancer of the subject is breast cancer is included into the evaluation result. In this embodiment, there is an additional advantage that the risk of suffering from cancer can be notified to the subject while designating the type of cancer as "breast cancer".

(14) In a further preferred embodiment of the cancer risk evaluation system according to the second aspect of the present invention, in a case where the age data and the concentration data of the 10 elements of Na, P, S, Ca, Fe, Cu, Zn, As, Cs, and Ag which are selected from the 17 elements used as the set of evaluation elements are judged significant for discrimination based on the correlation which is operated by the evaluation result operation section, and the subject is female, an estimate that a type of cancer of the subject is colorectal cancer is included into the evaluation result. In this embodiment, there is an additional advantage that the risk of suffering from cancer can be notified to the subject while designating the type of cancer as "colorectal cancer".

## ADVANTAGEOUS EFFECTS OF THE INVENTION

With the cancer risk evaluation method according to the first aspect of the present invention and the cancer risk evaluation system according to the second aspect of the present invention, there are advantageous effects that (a) the risk of suffering from cancer of a subject can be estimated with high accuracy, the disadvantages of early degeneration and high cost that arise in the case where the in-blood amino acid concentrations are utilized do not occur, and which site of cancer a subject has can be estimated; and (b) this method and this system can be applied easily to group or mass examinations.

BRIEF DESCRIPTION OF THE DRAWINGS

Fig. 1 is a flowchart showing the basic principle of a cancer risk evaluation method according to the present invention.

Fig. 2 is a functional block diagram showing the basic structure of a cancer risk evaluation system according to the present invention.

Fig. 3 is a table showing the distinction of sex and the age distribution of all subjects who provided their serums in examples 1 to 4 of the cancer risk evaluation method according to the present invention.

Fig. 4 is a table showing the breakdown of all subjects (a cancer patient group and a control group) who provided their serums and the site (type) of cancer of the cancer patients in the examples 1 to 4 of the cancer risk evaluation method according to the present invention.

Fig. 5 is a table showing the concentration data of 17 elements used as a set of evaluation elements and the age data in the examples 1 to 4 of the cancer risk evaluation method according to the present invention, in which the elements having a positive or negative correlation with respect to the cancer risk are indicated (which are judged significant by discriminant analysis and logistic regression analysis).

Fig. 6 shows a table indicating the numbers and percentages of the cancer patients and the controls (the case group and the control group) who provided their serums and a table indicating the fundamental statistics of these two groups (all the subjects) in the example 1 (pancreatic cancer, male) of the cancer risk evaluation method according to the present invention.

Fig. 7 shows a table indicating the variables contained in the discriminant function used and a table indicating the discriminant coefficients of these variables in the example 1 (pancreatic cancer, male) of the cancer risk evaluation method according to the present invention.

Fig. 8 shows a table indicating the centroids of the cancer patients (the case group) and the controls (the control group) and a table indicating the discrimination result in the example 1 (pancreatic cancer, male) of the cancer risk evaluation method according to the present invention.

Fig. 9 shows a table indicating the numbers and percentages of the cancer patients and the controls (the case group and the control group) who provided their serums and a table indicating the fundamental statistics of these two groups (all the subjects) in the example 2 (prostate cancer, male) of the cancer risk evaluation method according to the present invention.

Fig. 10 shows a table indicating the variables contained in the discriminant function used and a table indicating the discriminant coefficients of these variables in the example 2 (prostate cancer, male) of the cancer risk evaluation method according to the present invention.

Fig. 11 shows a table indicating the centroids of the cancer patients (the case group) and the controls (the control group) and a table indicating the discrimination result in the example 2 (prostate cancer, male) of the cancer risk evaluation method according to the present invention.

Fig. 12 shows a table indicating the numbers and percentages of the cancer patients and the controls (the case group and the control group) who provided their serums and a table indicating the fundamental statistics of these two groups (all the subjects) in the example 3 (colorectal cancer, male) of the cancer risk evaluation method according to the present invention.

Fig. 13 shows a table indicating the variables contained in the discriminant function used and a table indicating the discriminant coefficients of these variables in the example 3 (colorectal cancer, male) of the cancer risk evaluation method according to the present invention.

Fig. 14 shows a table indicating the centroids of the cancer patients (the case group) and the controls (the control group) and a table indicating the discrimination result in the example 3 (colorectal cancer, male) of the cancer risk evaluation method according to the present invention.

Fig. 15 shows a table indicating the numbers and percentages of the cancer patients and the controls (the case group and the control group) who provided their serums and a table indicating the fundamental statistics of these two groups (all the subjects) in the example 4 (endometrial cancer, male) of the cancer risk evaluation method according to the present invention.

Fig. 16 shows a table indicating the variables contained in the discriminant function used and a table indicating discriminant coefficients of these variables in the example 4 (endometrial cancer, male) of the cancer risk evaluation method according to the present invention.

Fig. 17 shows a table indicating the centroids of the cancer patients (the case group) and the controls (the control group) and a table indicating the discrimination result in the example 4 (endometrial cancer, male) of the cancer risk evaluation method according to the present invention.

Fig. 18 shows a table indicating the numbers and percentages of the cancer patients and the controls (the case group and the control group) who provided their serums and a table indicating the fundamental statistics of these two groups (all the subjects) in the example 5 (breast cancer, male) of the cancer risk evaluation method according to the present invention.

Fig. 19 shows a table indicating the variables contained in the discriminant function used and a table indicating the discriminant coefficients of these variables in the example 5 (breast cancer, male) of the cancer risk evaluation method according to the present invention.

Fig. 20 shows a table indicating the centroids of the cancer patients (the case group) and the controls (the control group) and a table indicating the discrimination result in the example 5 (breast cancer, male) of the cancer risk evaluation method according to the present invention.

Fig. 21 shows a table indicating the numbers and percentages of the cancer patients and the controls (the case group and the control group) who provided their serums and a table indicating the fundamental statistics of these two groups (all the subjects) in the example 6 (colorectal cancer, male) of the cancer risk evaluation method according to the present invention.

Fig. 22 shows a table indicating the variables contained in the discriminant function used and a table indicating the discriminant coefficients of these variables in the example 6 (colorectal cancer, male) of the cancer risk evaluation method according to the present invention.

Fig. 23 shows a table indicating the centroids of the cancer patients (the case group) and the controls (the control group) and a table indicating the discrimination result in the example 6 (colorectal cancer, male) of the cancer risk evaluation method according to the present invention.

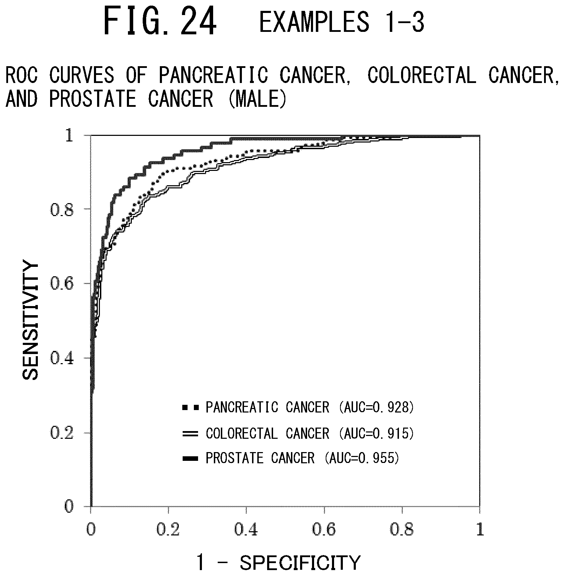

Fig. 24 is a graph showing the results of ROC analysis of the pancreatic, colorectal, and prostate cancer patients (male) which are obtained in the examples 1 to 3 of the cancer risk evaluation method according to the present invention.

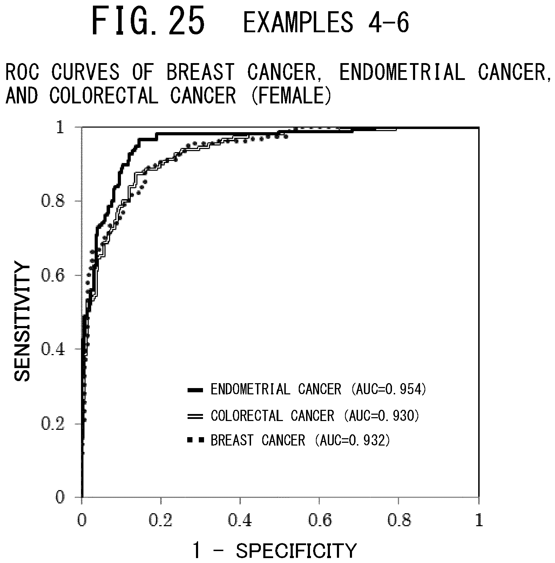

Fig. 25 is a graph showing the results of ROC analysis of the breast, endometrial, and colorectal cancer patients (female) which are obtained in the examples 4 to 6 of the cancer risk evaluation method according to the present invention.

Fig. 26 is a graph showing the relationship between the discriminant score and the cancer probability in the example 1 of the cancer risk evaluation method according to the present invention.

Fig. 27 is a graph showing the relationship between the discriminant score and the cancer probability in the example 2 of the cancer risk evaluation method according to the present invention.

Fig. 28 is a graph showing the relationship between the discriminant score and the cancer probability in the example 3 of the cancer risk evaluation method according to the present invention.

Fig. 29 is a graph showing the relationship between the discriminant score and the cancer probability in the example 4 of the cancer risk evaluation method according to the present invention.

Fig. 30 is a graph showing the relationship between the discriminant score and the cancer probability in the example 5 of the cancer risk evaluation method according to the present invention.

Fig. 31 is a graph showing the relationship between the discriminant score and the cancer probability in the example 6 of the cancer risk evaluation method according to the present invention.

EMBODIMNENTS FOR CARRYING OUT THE INVENTION

Preferred embodiments of the present invention will be described below in detail while referring to the drawings attached.

[Basic Principle of Cancer Risk Evaluation Method of Invention]

The inventors developed the cancer evaluation method that utilizes the correlations between the onset of cancer and the concentrations (contents) of elements contained in a human serum as a novel screening method for cancers, as disclosed in the aforementioned Patent Literature 2. Based on further findings obtained in the development process of the aforementioned cancer evaluation method, the inventers conducted earnest researches furthermore and as a result, created the present invention.

In the present invention, first, serums that belong to cancer patients (a case group) and those that belong to controls (a control group) are classified into two classes at random in accordance with sex, age class, and site, in which one of the two classes is termed "testing serums" and the other thereof is termed "evaluating serums". Next, the concentrations of in-serum elements are measured using the testing serums and then, the concentrations thus measured are analyzed statistically to form a discriminant. Subsequently, age data and concentration data of the evaluating serums are applied to the discriminant thus formed, thereby generating an indicator of whether or not a subject suffers from any type of cancer. An estimation of which site of cancer the subject has is contained in this indicator according to the necessity.

Next, the cancer risk evaluation method according to the present invention will be explained in detail below.

First, the inventors conducted a preliminary treatment in the following way, thereby finding an optimal measuring condition for the concentration measurement of elements contained in a serum.

Nitric acid was mixed with the testing serums (which contain both of the serums that belong to the case group and those that belong to the control group) and then, the mixture thus generated was heated at a temperature between 180°C and 200°C in a sealed pressure vessel having low metal contamination to decompose proteins and amino acids contained in the mixture. This was to conduct a pretreatment in such a way as not to interfere with the concentration measurement of the elements. Subsequently, the mixture was diluted to a predetermined concentration using ultrapure water having no metal contamination, generating a processing liquid. Then, the concentrations of the 75 elements contained in the processing liquid thus generated were measured using the ICP Mass Spectrometry. Using the result thus obtained, an optimal measuring condition for the concentration measurement of the elements contained in the testing serums was found.

To conduct the concentration measurement of various types of elements, Inductively-Coupled Plasma Optical Emission Spectroscopy (ICP-OES), Inductively-Coupled Plasma Mass Spectroscopy (ICP-MS), Atomic Absorption Spectrometry (AAS), X-Ray Fluorescence analysis (XRF) and so on can be used in addition to ICP Mass Spectrometry. The reason why the inventors chose ICP Mass Spectrometry is that ICP Mass Spectrometry is recognized to be the simplest way where the quantitativity in measurement result is strict. Accordingly, if this condition is changed, and/or any other analyzing method that is more preferred is developed, it is needless to say that any other method than ICP Mass Spectrometry may be used for this purpose.

Using the same testing serums (which contain both of the serums that belong to the case group and those that belong to the control group) under the optimal measuring condition thus found, the contents of the 75 elements contained in the said testing serums were measured using ICP Mass Spectrometry. Thereafter, the difference of the concentration data of the elements thus measured between the case group and the control group was analyzed statistically. In this analysis, to clarify the elements that are concerned with the difference between the case group and the control group and to find the risk (probability) of having cancer, discriminant analysis and binomial logistic regression analysis were used. At this stage, the combinations of the elements are taken into consideration, and a combination of elements that maximizes the difference between the elements, in other words, a combination of elements that distinguishes between the case group and the control group most favorably was explored using a computer while changing the combinations of the elements many times over. As a result, it was found that the discriminant ability was the highest in the case where a combination of 17 elements of Na, Mg, P, S, K, Ca, Fe, Cu, Zn, Se, Rb, Sr, As, Mo, Cs, Co, and Ag was used. Accordingly, the inventors decided that the combination of these 17 elements was used as a "set of evaluation elements" in the present invention.

After the "set of evaluation elements" was determined in the aforementioned manner, the concentrations of the in-serum elements are measured using the same testing serums. Then, discriminant analysis is conducted for the element concentrations thus measured, thereby forming a discriminant. When a discriminant is formed in this way, a discriminant value is calculated by applying age data and element concentration data of the evaluating serums to the discriminant thus formed and as a result, an indicator of whether or not a subject suffers from any type of cancer is obtained. Moreover, the risk (probability) of having cancer of the subject is found by conducting binomial logistic regression analysis using the discriminant value thus calculated. Furthermore, which site of cancer the subject has can be estimated also by knowing which of the concentration data of the aforementioned 17 elements used as the set of evaluation elements is/are significant for discrimination.

The details of the discriminant analysis and the binomial logistic regression analysis described above will be explained below.

First, discriminant analysis for the case group and the control group was conducted with respect to the 17 elements (Na, Mg, P, S, K, Ca, Fe, Cu, Zn, Se, Rb, Sr, As, Mo, Cs, Co, and Ag) to be measured as the "set of evaluation elements". Concretely speaking, a test (t-test) for the difference between the population means of the case group and the control group was carried out. This was to search to what degree the discrimination between these two groups is affected by these 17 elements. In the result of this test, a difference was observed between these two groups with respect to the respective elements individually; however, the relationships among these elements were ignored in this analysis and therefore, this analysis included many problems if used for the purpose of evaluating the risk of disease. To solve these problems, it was necessary to conduct analysis using multivariate analysis which is capable of considering the relationships among the elements, i.e., discriminant analysis.

Accordingly, next, a discriminant function was obtained in the following way. This was to analyze the concentration balance (correlations) among the elements. The concentrations of the individual elements included personal differences and thus, they were difficult to be used as an indicator. For this reason, the correlations of the concentrations among the elements needed to be found.

A discriminant function can be expressed in the following equation (1).

$$\text{Discriminant Value (D)} = \text{Function (F) (Explanatory Variables 1 to n, Discriminant Coefficients)} \quad (1)$$

(N is an integer equal to or greater than 2.)

Taking the weights (the influences on discrimination) of the respective explanatory variables 1 to n into consideration, the equation (1) can be written as the following equation (2).

$$\text{Discriminant Value (D)} = \text{(Discriminant Coefficient 1)} \times \text{(Explanatory Variable 1)} + \text{(Discriminant Coefficient 2)} \times \text{(Explanatory Variable 2)} + \cdots \text{(Discriminant Coefficient n)} \times \text{(Explanatory Variable n)} + \text{Constant} \quad (2)$$

Here, the concentrations of the 17 elements (Na, Mg, P, S, K, Ca, Fe, Cu, Zn, Se, Rb, Sr, As, Mo, Cs, Co, and Ag), which were chosen from the result of the test (t-test) for the difference between the population means of the two groups, and the age of the subject are defined as the explanatory variables and at the same time, the discriminant coefficients are used as the weights for these explanatory variables. As a result, a discriminant function is obtained. A desired discriminant function can be easily obtained by inputting the concentration values (the concentration data) of these 17 elements and the age of the subject (the age data) into a known discriminant analysis program.

When discriminant value (discriminant score) (D) calculated in this way is equal to 0 or less, it is judged that the subject belongs to the case group, and when the discriminant value (D) is equal to 0 or greater, it is judged that the subject belongs to the control group.

Next, to obtain the probability that the subject belongs to the case group or the control group, the binomial logistic regression analysis is carried out to obtain an incidence. The incidence is given by the following equation (3) using the discriminant value (D) which is obtained in the aforementioned discriminant analysis.

$$\text{Incidence} = 1 / [1 + \exp(-\text{Discriminant Value})] \quad (3)$$

Since the incidence can be obtained using the equation (3), the probability that the subject belongs to the case group also can be found. This means that the subject can know his/her own current risk of suffering from cancer as the probability.

As a result of the discriminant analysis, it was found that the discriminant ability was the highest when the aforementioned 17 elements (Na, Mg, P, S, K, Ca, Fe, Cu, Zn, Se, Rb, Sr, As, Mo, Cs, Co, and Ag) were used.

In the cancer risk evaluation method according to the present invention, the 17 elements (Na, Mg, P, S, K, Ca, Fe, Cu, Zn, Se, Rb, Sr, As, Mo, Cs, Co, and Ag) that were specified through the aforementioned preliminary treatment are designated as the set of evaluation elements and then, the concentrations of these 17 elements contained in the serum of a subject are measured, thereby obtaining an indicator of whether or not the subject suffers from any type of cancer.

With the cancer evaluation method according to the present invention, as shown in Fig. 1, first, a serum sample 2 that has been collected from a subject is put into a test tube 1 and then, the sample 2 is placed in an analyzing apparatus and analyzed, thereby measuring the concentrations of the predetermined elements (the set of evaluation elements) in the serum (Step S1). The elements whose concentrations are to be measured here are the aforementioned 17 elements (Na, Mg, P, S, K, Ca, Fe, Cu, Zn, Se, Rb, Sr, As, Mo, Cs, Co, and Ag).

Next, the in-serum concentration data of the set of evaluation elements obtained in the step S1 are applied to a predetermined discriminant function or functions (which is/are obtained by the aforementioned discriminant analysis) to conduct an operation (Step S2).

Finally, based on the operation result obtained in the step S2, an indicator of whether or not the subject from which the serum sample 2 has been collected suffers from any type of cancer is generated. As a result, a desired evaluation result about the presence or absence of suffering from cancer is obtained (Step S3).

With the cancer evaluation method according to the present invention, as explained above, in the step S2 of operating the correlation, the concentration data of the set of evaluation elements (Na, Mg, P, S, K, Ca, Fe, Cu, Zn, Se, Rb, Sr, As, Mo, Cs, Co, and Ag) contained in the serum which is taken from the subject and the age data of the subject are applied to the discriminant function or functions for discriminating which of the case group and the control group the subject belongs to, thereby operating the correlation among the concentrations of the set of evaluation elements in the serum.

Moreover, in the step S3 of obtaining an indicator, the indicator of whether or not the subject suffers from any type of cancer is obtained based on the correlation which is operated in the step S2. The indicator is generated based on the discriminant score or scores which is/are calculated by applying the concentration data and the age data to the discriminant function or functions which is/are used in the step S2.

Accordingly, the risk of suffering from cancer of the subject can be estimated with high accuracy and at the same time, the disadvantages of early degeneration and high cost that arise in the case where the in-blood amino acid concentrations are utilized do not occur.

Furthermore, it is known which of the concentration data of the aforementioned 17 elements (Na, Mg, P, S, K, Ca, Fe, Cu, Zn, Se, Rb, Sr, As, Mo, Cs, Co, and Ag) used as the set of evaluation elements is/are significant for discrimination in the step S2 of operating the correlation, and the one or more elements which is/are judged significant for discrimination is/are changed according to the type of cancer. As a result, which site of cancer the subject has can be estimated also.

Furthermore, which of the case group and the control group the subject belongs to can be discriminated by automatic operation with a computer using the concentration data of the set of evaluation elements in the serum which is taken from the subject and the age data of the subject. Accordingly, the discrimination can be performed easily and quickly even if the number of the subjects is large. This means that the method according to the present invention is easily applicable to group or mass examinations.

[Basic structure of Cancer risk evaluation system of invention]

Next, a cancer risk evaluation system according to the present invention will be explained below.

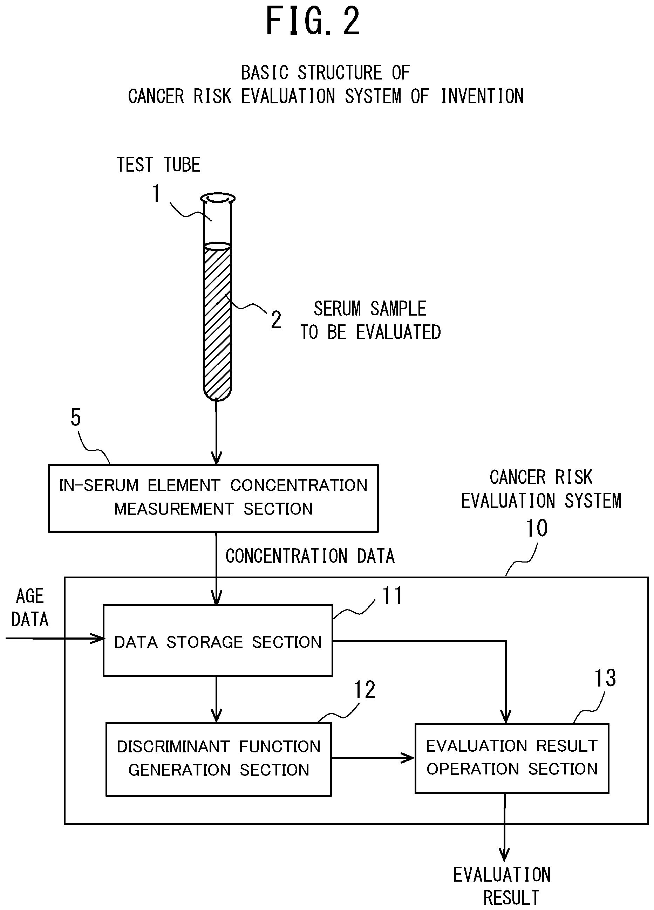

The basic structure of the cancer risk evaluation system 10 of the present invention is shown in Fig 2. The cancer risk evaluation system 10, which is a system for carrying out the aforementioned cancer risk evaluation method of the present invention, comprises a data storage section 11, a discriminant function generation section 12, and an evaluation result operation section 13, as seen from Fig. 2.

An in-serum element concentration measurement section 5 is provided outside the cancer risk evaluation system 10, in which the in-serum concentrations of the set of evaluation elements (Na, Mg, P, S, K, Ca, Fe, Cu, Zn, Se, Rb, Sr, As, Mo, Cs, Co, and Ag) are measured using a serum sample 2 that has been collected from a subject and that has been put into a test tube 1. The concentration data of the set of evaluation elements thus obtained in the in-serum element concentration measurement section 5 are supplied to the data storage section 11. The age data of the subject also is stored in the data storage section 11. As the in-serum element concentration measurement section 5, for example, a known ICP mass spectrometer is used.

The data storage section 11 is a section for storing the concentration data of the set of evaluation elements obtained in the in-serum element concentration measurement section 5 and the age data, which is usually formed by a known storage device.

The discriminant function generation section 12 is a section for generating a discriminant function or functions that is/are used for the operation in the evaluation result operation section 13, which is usually formed to include a known program.

The evaluation result operation section 13 conducts the operation using a predetermined method. Based on the operation result outputted by the evaluation result operation section 13, a desired evaluation result is obtained, in other words, the risk of suffering from cancer of the subject is evaluated.

When the aforementioned cancer risk evaluation method according to the present invention is carried out with the cancer risk evaluation system 10, the risk of suffering from cancer is calculated using, for example, pattern analysis of the in-serum concentrations of the set of evaluation elements, and the result that the possibility of having cancer is expressed stochastically based on the said risk is presented. Concretely speaking, serums (each of which is 0.5 cc in volume, for example) are collected at physical checkups which are conducted in medical institutions or diagnosis institutions and then, they are subjected to concentration measurement of the set of specific evaluation elements (Na, Mg, P, S, K, Ca, Fe, Cu, Zn, Se, Rb, Sr, As, Mo, Cs, Co, and Ag) at inspection agencies. Thereafter, based on the concentration data of the set of evaluation elements thus measured at the inspection agencies and the age data of the subjects, the risk of suffering from cancer is calculated at an institution like, for example, a risk evaluation center (provisional name). The calculation result of the risk thus obtained is delivered to blood collection agencies and then, sent to each of the medical examinees from the blood collection agencies. When the examinees are suspected to have cancer, the blood collection agencies recommend them to receive "existing cancer examination". The personal information is systemized so as not to reach the inspection agencies and the risk evaluation center through the encryption or consecutive numbering which is executed at the blood collection agencies.

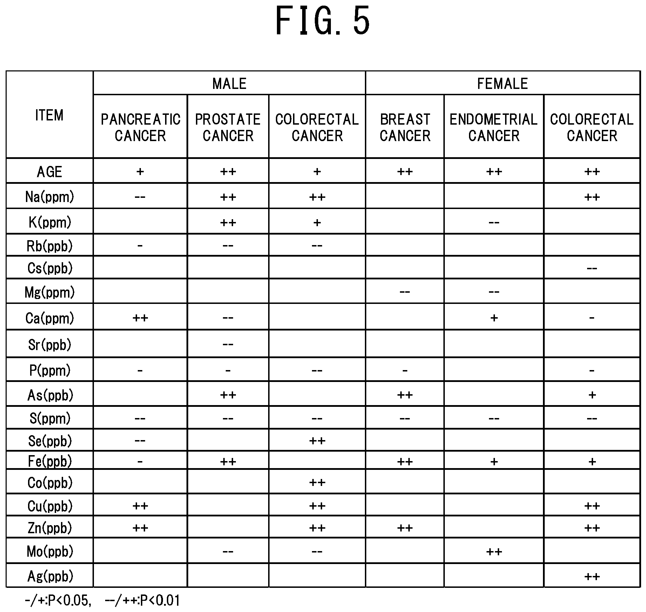

From the results of the examples 1 to 6 which will be described below, it was shown that the risks of suffering from male pancreatic cancer, male prostate cancer, male colorectal cancer, female endometrial cancer, female breast cancer, and female colorectal cancer were able to be calculated using the concentration data of the in-serum 17 elements and the age data. The reason why the risks of suffering from different sites of cancer can be calculated using the discriminant scores which are measured through one-time blood collection is that the elements that are significantly concerned with the discrimination are different in accordance with the distinction of sex and the site of cancer, as shown in Fig. 5. This figure shows that the common items for these types of cancer are the age and sulfur (S) and the risks of all of these types of cancer increase with aging, and that the said risks become higher when the in-serum concentration of sulfur decreases. However, it is apparent that the effects of the 16 elements in the serum excluding sulfur are different largely in accordance with the distinction of the site of cancer. It is inferred that such the differences make it possible to estimate the risks of suffering from different sites of cancer.

EXAMPLES

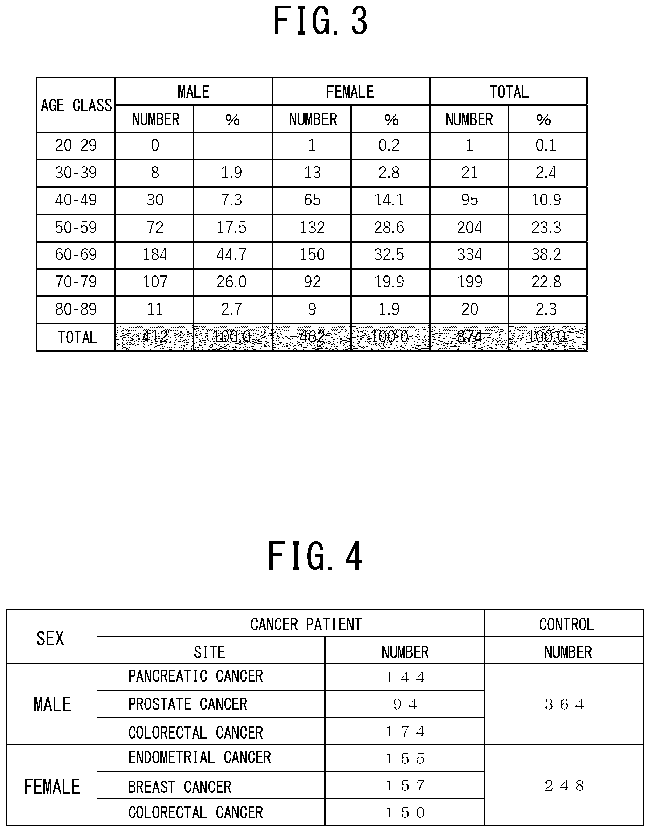

The present invention will be explained in more detail based on examples. The numbers of subjects whose risks of suffering from cancer are to be estimated are shown in Fig. 4. Specifically, the number of a male case group (male cancer patients) was 712 in total, in which 144 pancreatic cancer patients, 94 prostate cancer patients, and 174 colorectal cancer patients were included. The number of a male control group (controls) was 364. On the other hand, the number of a female case group (female cancer patients) was 462 in total, in which 155 endometrial cancer patients, 157 breast cancer patients, and 150 colorectal cancer patients were included. The number of a female control group (controls) was 248.

Moreover, the subjects who belong to one of the male and female case groups and the male and female control groups shown in Fig. 4 were classified into 7 age classes, i.e., the 20 to 29 age class, the 30 to 39 age class, the 40 to 49 age class, the 50 to 59 age class, the 60 to 69 age class, the 70 to 79 age class, and the 80 to 89 age class, as shown in Fig. 3.

The items shown in Fig. 5 affected the cancer risk evaluation. Specifically, regarding the male pancreatic cancer patients, the age data and the concentration data of the 9 elements of Na, P, S, Ca, Fe, Cu, Zn, Se, and Rb, which were selected from the 17 elements used as the set of evaluation elements, were judged significant for discrimination. Regarding the male prostate cancer patients, the age data and the concentration data of the 10 elements of Na, P, S, K, Ca, Fe, As, Sr, Rb, and Mo, which were selected from the 17 elements used as the set of evaluation elements, were judged significant for discrimination. Regarding the male colorectal cancer patients, the age data and the concentration data of the 10 elements of Na, P, S, K, Cu, Zn, Rb, Se, Mo, and Co, which were selected from the 17 elements used as the set of evaluation elements, were judged significant for discrimination. Regarding the female endometrial cancer patients, the age data and the concentration data of the 6 elements of Mg, S, K, Ca, Fe, and Mo, which were selected from the 17 elements used as the set of evaluation elements, were judged significant for discrimination. Regarding the female breast cancer patients, the age data and the concentration data of the 6 elements of Mg, P, S, Fe, Zn, and Cs, which were selected from the 17 elements used as the set of evaluation elements, were judged significant for discrimination. Regarding the female colorectal cancer patients, the age data and the concentration data of the 10 elements of Na, P, S, Ca, Fe, Cu, Zn, As, Cs, and Ag, which were selected from the 17 elements used as the set of evaluation elements, were judged significant for discrimination.

EXAMPLE 1

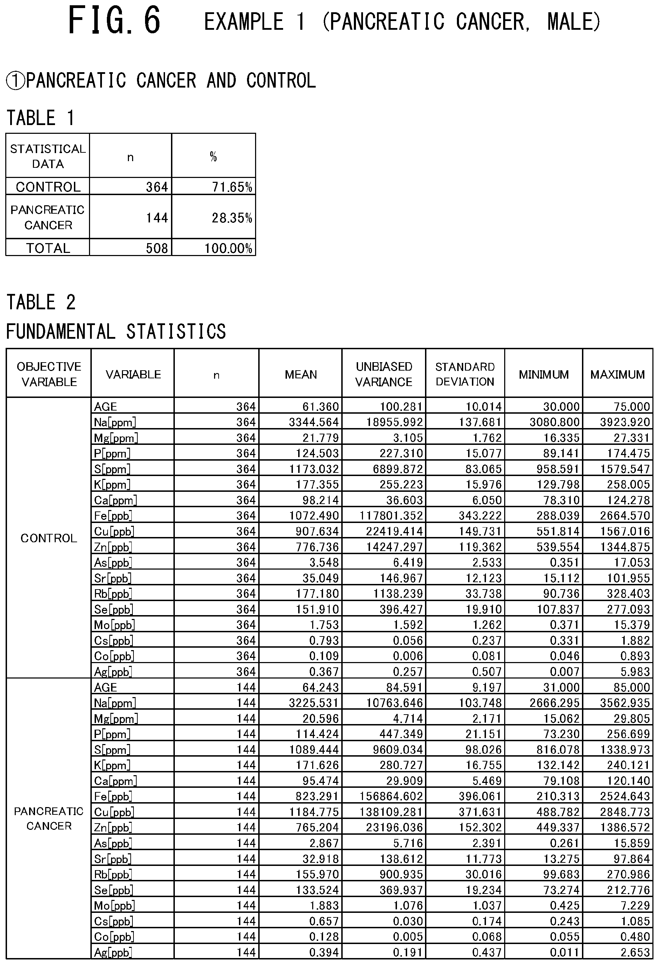

In the example 1, the risk of suffering from male pancreatic cancer was estimated. The subjects whose cancer risk was to be estimated in this example were 144 subjects who belonged to the case group (male pancreatic cancer patients) and 364 subjects who belonged to the male control group (controls), as shown in the table 1 of Fig. 6. The serums of these subjects were used as the evaluation targets. The data used in this evaluation were the age data of the subjects and the concentration data of the 17 elements (Na, Mg, P, S, K, Ca, Fe, Cu, Zn, Se, Rb, Sr, As, Mo, Cs, Co, and Ag) which were used as the set of evaluation elements.

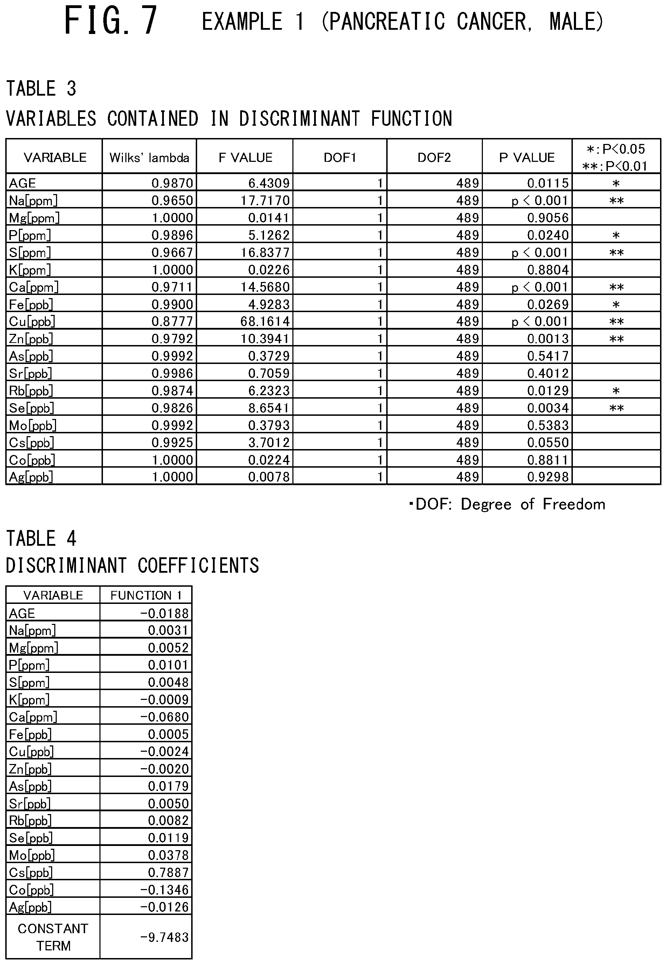

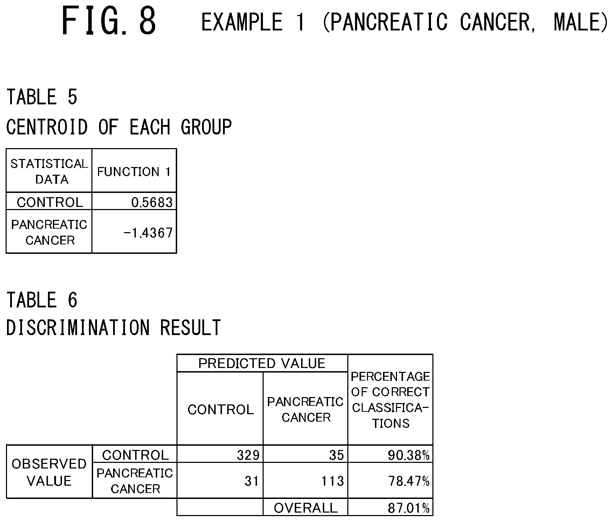

The mean value, the standard deviation, the maximum value, and the minimum value of each of the items are shown in the table 2 of Fig. 6, in which the subjects are classified into the case group (pancreatic cancer patients) and the control group (controls). As a result of discriminant analysis, as shown in the table 3 of Fig. 7, it was found that the age data and the 9 elements of Na, P, S, Ca, Fe, Cu, Zn, Rb, and Se were judged significant for discrimination. The discriminant coefficients and the constant term of the discriminant used in this discriminant analysis were shown in the Table 4 of Fig. 7. As shown in the table 5 of Fig. 8, it can be concluded that the element whose discriminant coefficient has a plus (+) sign is strongly relevant to the control group and the element whose discriminant coefficient has a minus (-) sign is strongly relevant to the case group (having pancreatic cancer) from the centroid values of the case group (male pancreatic cancer patients) and the control group (controls).

The discrimination result is shown in the table 6 of Fig. 8. According to this result, the 113 cases out of the 144 cases belonging to the case group were correctly classified (Sensitivity: 78.5%), and the 329 samples out of the 364 samples belonging to the control group were correctly classified (Specificity: 90.4%). Accordingly, it was indicated that the accuracy rate had a high value of 87.0%.

When calculating the Area Under the Curve (AUC) from the ROC curve which was obtained from the discriminant analysis, a high value of 0.928 was obtained, as shown in Fig, 24.

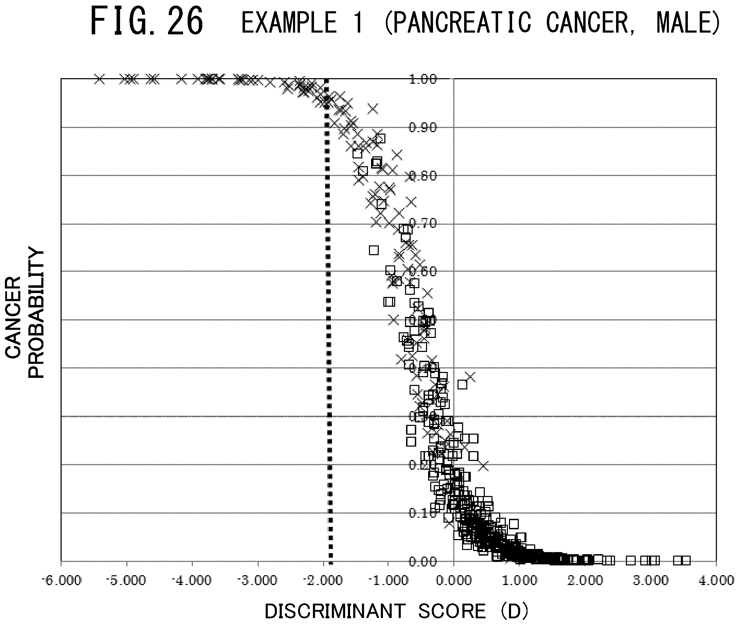

Using these results, the discriminant score was calculated by inputting the concentration data of the 17 trace elements (the set of evaluation elements) (Na, Mg, P, S, K, Ca, Fe, Cu, Zn, Se, Rb, Sr, As, Mo, Cs, Co, and Ag) contained in the serums and the age data of the subjects into the aforementioned discriminant and then, the risk (probability) of suffering from pancreatic caner was calculated using the discriminant score thus calculated. The result of this calculation is shown in Fig. 26.

As seen from Fig. 26, in the case of male pancreatic cancer, as the discriminant score having a negative value increases, the risk rises. Specifically, it can be interpreted that acquiring pancreatic cancer is estimated with a probability of 95% or higher when the value of the discriminant score is approximately equal to -1.8 or lower.

EXAMPLE 2

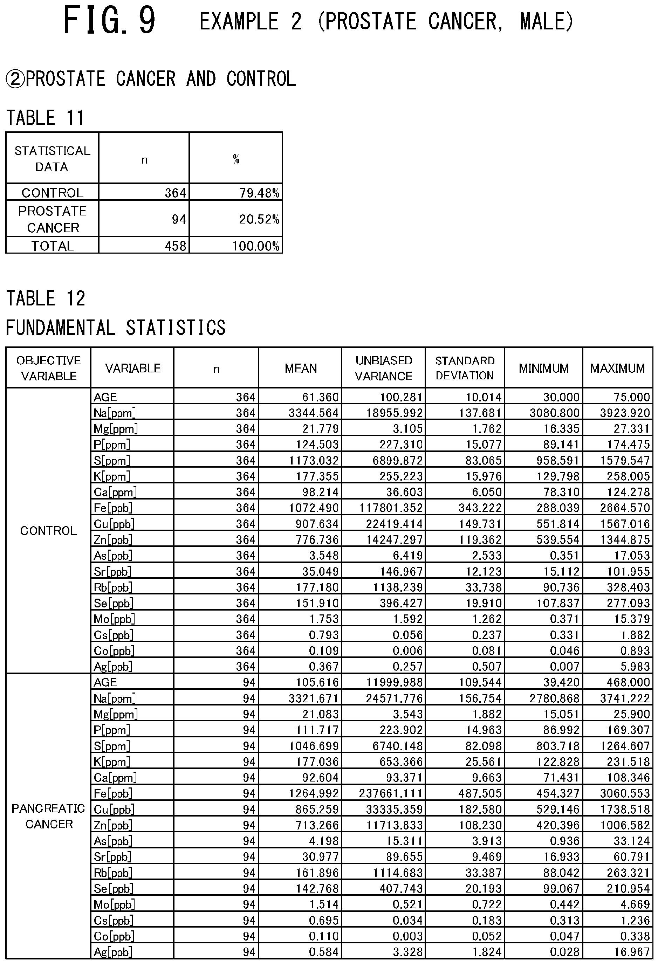

In the example 2, the risk of suffering from male prostate cancer was estimated. The subjects whose cancer risk was to be estimated in this example were 94 subjects who belonged to the case group (male prostate cancer patients), and 364 subjects who belonged to the male control group (controls) which is the same as the example 1, as shown in the table 11 of Fig. 9. The serums of these subjects were used as the evaluation targets. The data used in this evaluation were the age data of the subjects and the concentration data of the 17 elements which were used as the set of evaluation elements in the example 1.

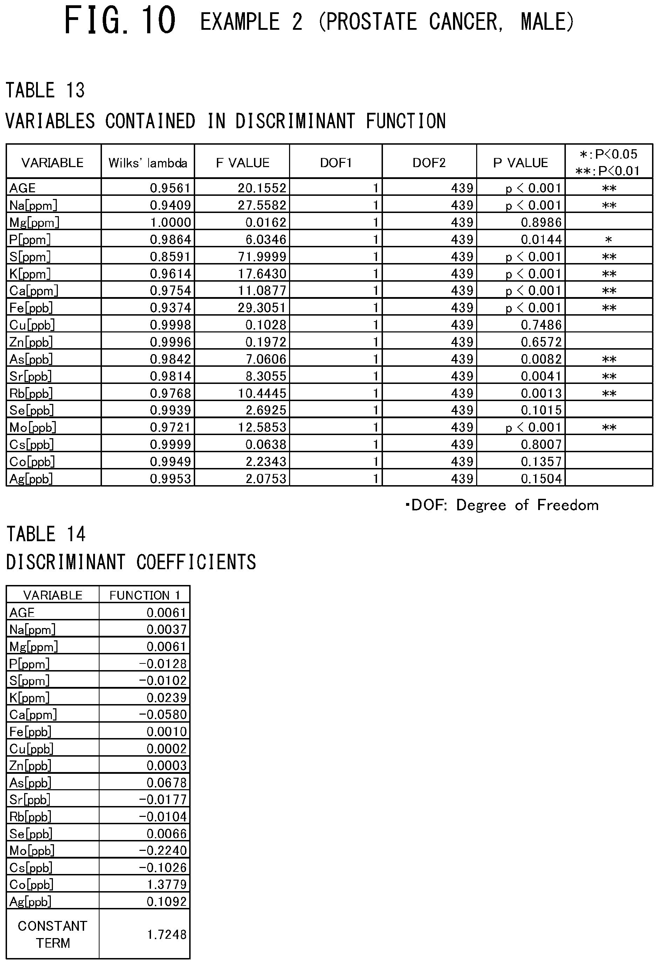

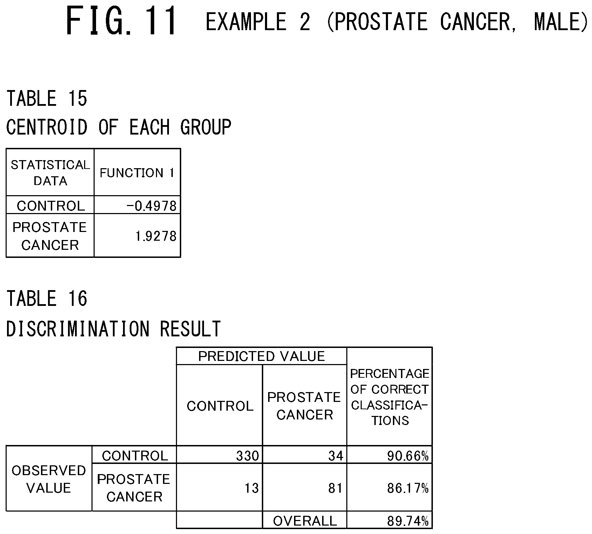

The mean value, the standard deviation, the maximum value, and the minimum value of each of the items are shown in the table 12 of Fig. 9, in which the subjects are classified into the case group (prostate cancer patients) and the control group (controls). As a result of discriminant analysis, as shown in the table 13 of Fig. 10, it was found that the age data and the 10 elements of Na, P, S, K, Ca, Fe, As, Sr, Rb, and Mo were judged significant for discrimination. The discriminant coefficients and the constant term of the discriminant used in this discriminant analysis were shown in the Table 14 of Fig. 10. As shown in the table 15 of Fig. 11, it can be concluded that the element whose discriminant coefficient has a plus (+) sign is strongly relevant to the case group (having prostate cancer) and the element whose discriminant coefficient has a minus (-) sign is strongly relevant to the control group (controls) from the centroid values of the case group (male prostate cancer patients) and the control group (controls).

The discrimination result is shown in the table 16 of Fig. 11. According to this result, the 81 cases out of the 94 cases belonging to the case group were correctly classified (Sensitivity: 86.2%), and the 330 samples out of the 364 samples belonging to the control group were correctly classified (Specificity: 90.7%). Accordingly, it was indicated that the accuracy rate had a high value of 89.7%.

When calculating the Area Under the Curve (AUC) from the ROC curve which was obtained from the discriminant analysis, a high value of 0.955 was obtained, as shown in Fig, 24.

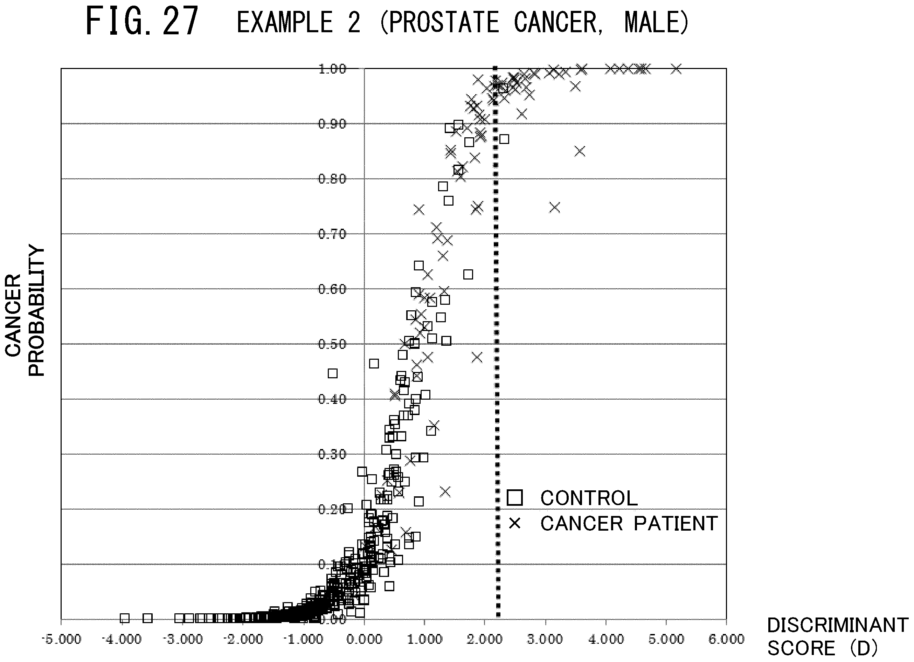

Using these results, similar to the example 1, the discriminant score was calculated by inputting the concentration data of the 17 trace elements (the set of evaluation elements) contained in the serums and the age data of the subjects into the aforementioned discriminant and then, the risk (probability) of suffering from prostate cancer was calculated using the discriminant score thus calculated. The result of this calculation is shown in Fig. 27.

As seen from Fig. 27, in the case of male prostate cancer, as the discriminant score having a positive value increases, the risk rises. Specifically, it can be interpreted that acquiring prostate cancer is estimated with a probability of 95% or higher when the value of the discriminant score is approximately equal to 2.3 or higher.

EXAMPLE 3

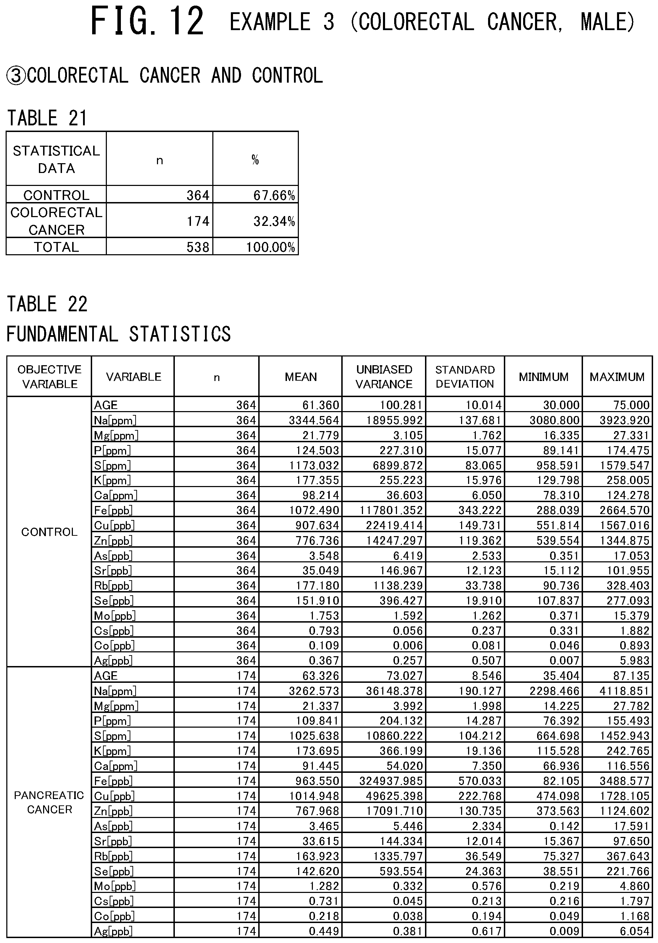

In the example 3, the risk of suffering from male colorectal cancer was estimated. The subjects whose cancer risk was to be estimated in this example were 174 subjects who belonged to the case group (male colorectal cancer patients), and 364 subjects who belonged to the male control group (controls) which is the same as the example 1, as shown in the table 21 of Fig. 12. The serums of these subjects were used as the evaluation targets. The data used in this evaluation were the age data of the subjects and the concentration data of the 17 elements which were used as the set of evaluation elements in the example 1.

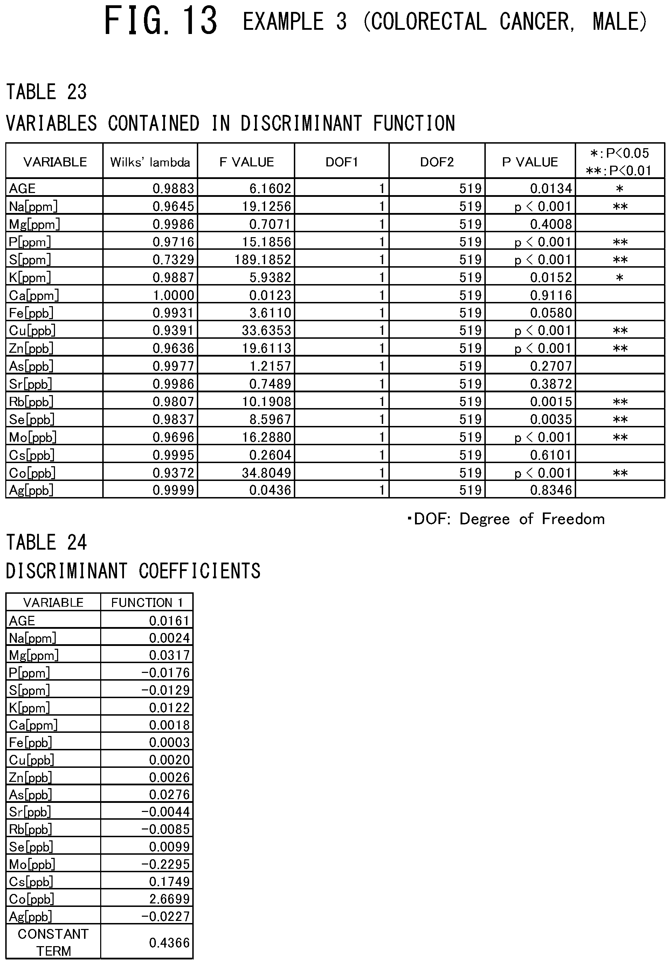

The mean value, the standard deviation, the maximum value, and the minimum value of each of the items are shown in the table 22 of Fig. 12, in which the subjects are classified into the case group (colorectal cancer patients) and the control group (controls). As a result of discriminant analysis, as shown in the table 23 of Fig. 13, it was found that the age data of the subjects and the 10 elements of Na, P, S, K, Cu, Zn, Rb, Se, Mo, and Co were judged significant for discrimination. The discriminant coefficients and the constant term of the discriminant used in this discriminant analysis were shown in the Table 24 of Fig. 13. As shown in the table 25 of Fig. 14, it can be concluded that the element whose discriminant coefficient has a plus (+) sign is strongly relevant to the casa group (colorectal cancer patients) and the element whose discriminant coefficient has a minus (-) sign is strongly relevant to the control group (controls) from the centroid values of the case group (male colorectal cancer patients) and the control group (controls).

The discrimination result is shown in the table 26 of Fig. 14. According to this result, the 152 cases out of the 174 cases belonging to the case group were correctly classified (Sensitivity: 87.4%), and the 338 samples out of the 364 samples belonging to the control group were correctly classified (Specificity: 92.9%). Accordingly, it was indicated that the accuracy rate had a high value of 87.0%.

When calculating the Area Under the Curve (AUC) from the ROC curve which was obtained from the discriminant analysis, a high value of 0.915 was obtained, as shown in Fig, 24.

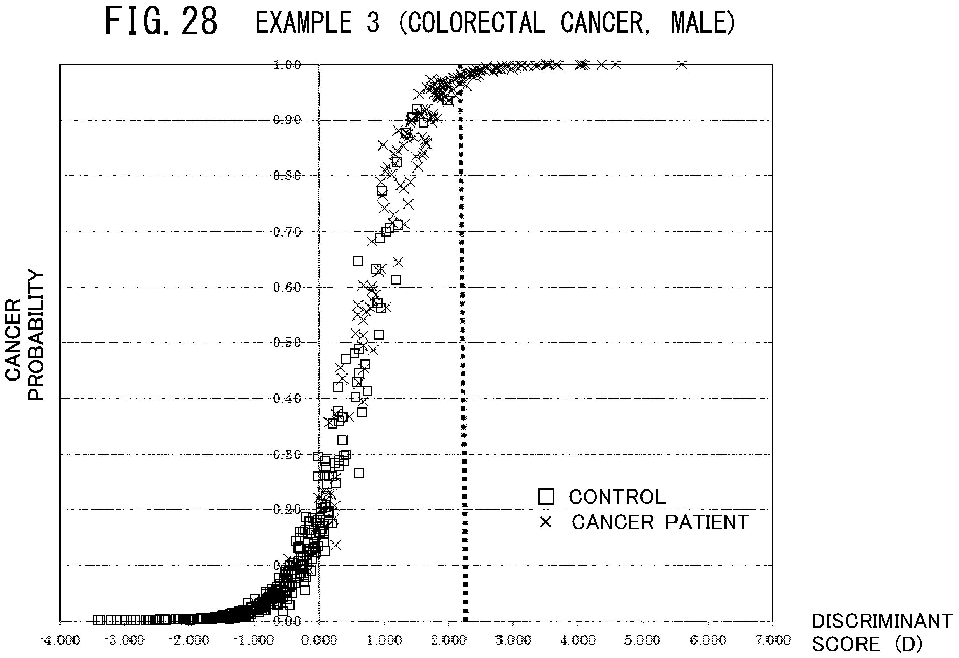

Using these results, similar to the example 1, the discriminant score was calculated by inputting the concentration data of the 17 trace elements (the set of evaluation elements) contained in the serums and the age data of the subjects into the aforementioned discriminant and then, the risk (probability) of suffering from colorectal cancer was calculated using the discriminant score thus calculated. The result of this calculation is shown in Fig. 28.

As seen from Fig. 28, in the case of male colorectal cancer, as the discriminant score having a positive value increases, the risk rises. Specifically, it can be interpreted that acquiring colorectal cancer is estimated with a probability of 95% or higher when the value of the discriminant score is approximately equal to 2.3 or higher.

EXAMPLE 4

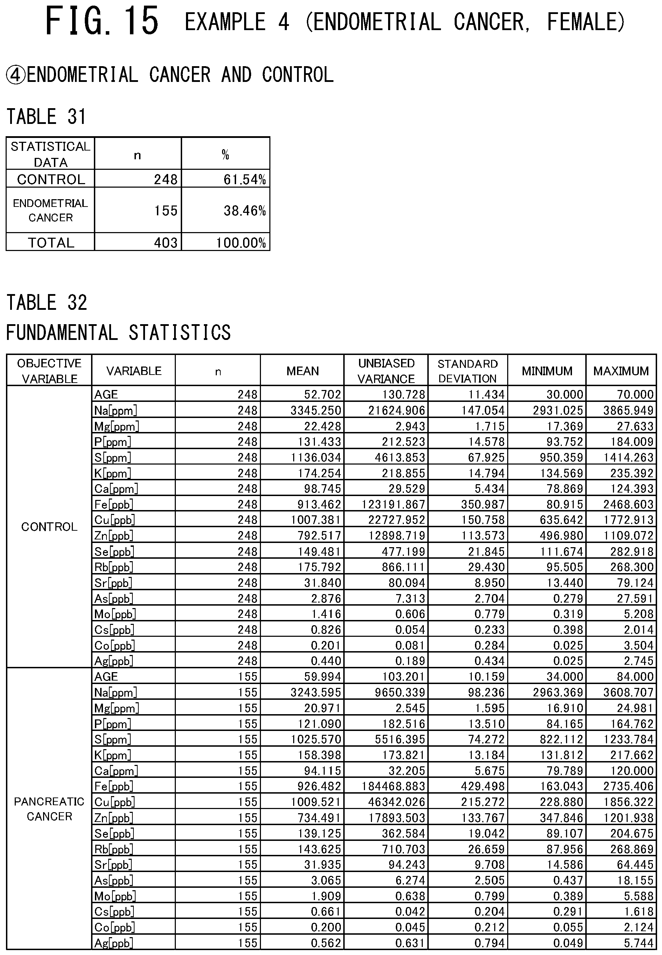

In the example 4, the risk of suffering from female endometrial cancer was estimated. The subjects whose cancer risk was to be estimated in this example were 155 subjects who belonged to the case group (female endometrial cancer patients) and 248 subjects who belonged to the female control group (controls), as shown in the table 31 of Fig. 15. The serums of these subjects were used as the evaluation targets. The data used in this evaluation were the age data of the subjects and the concentration data of the 17 elements which were used as the set of evaluation elements in the example 1.

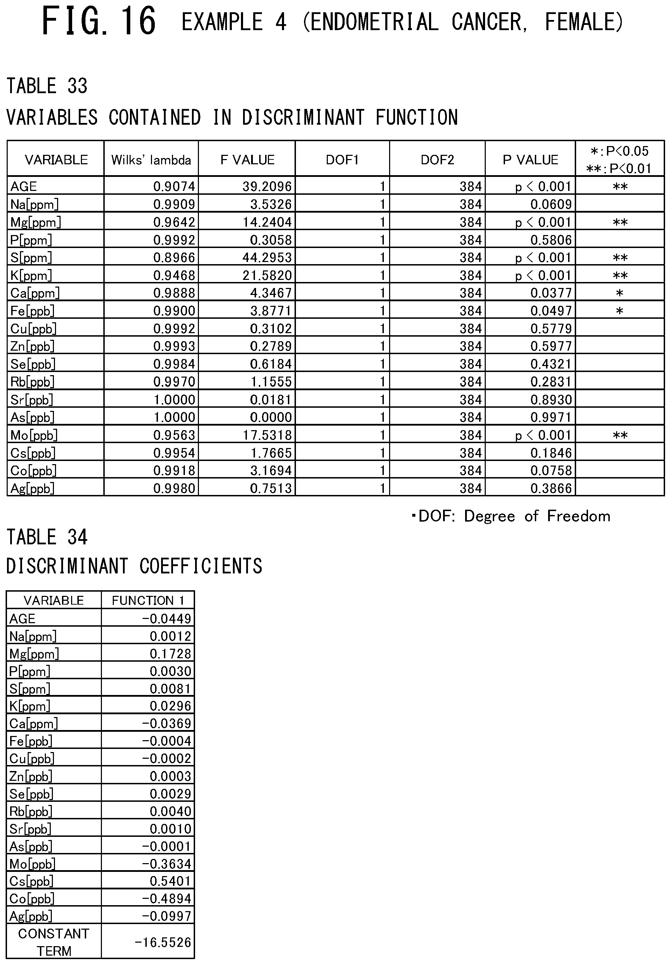

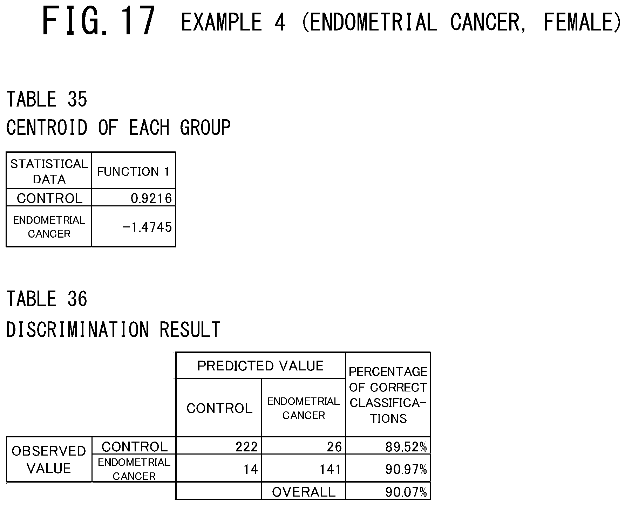

The mean value, the standard deviation, the maximum value, and the minimum value of each of the items are shown in the table 32 of Fig. 15, in which the subjects are classified into the case group (endometrial cancer patients) and the control group (controls). As a result of discriminant analysis, as shown in the table 33 of Fig. 16, it was found that the age data and the 6 elements of Mg, S, K, Ca, Fe, and Mo were judged significant for discrimination. The discriminant coefficients and the constant term of the discriminant used in this discriminant analysis were shown in the Table 34 of Fig. 16. As shown in the table 35 of Fig. 17, it can be concluded that the element whose discriminant coefficient has a plus (+) sign is strongly relevant to the control group (controls) and the element whose discriminant coefficient has a minus (-) sign is strongly relevant to the case group (having endometrial cancer) from the centroid values of the case group (female endometrial cancer patients) and the control group (controls).

The discrimination result is shown in the table 36 of Fig. 17. According to this result, the 141 cases out of the 155 cases belonging to the case group were correctly classified (Sensitivity: 91.0%), and the 222 samples out of the 248 samples belonging to the control group were correctly classified (Specificity: 89.5%). Accordingly, it was indicated that the accuracy rate had a high value of 90.1%.

When calculating the Area Under the Curve (AUC) from the ROC curve which was obtained from the discriminant analysis, a high value of 0.954 was obtained, as shown in Fig, 25.

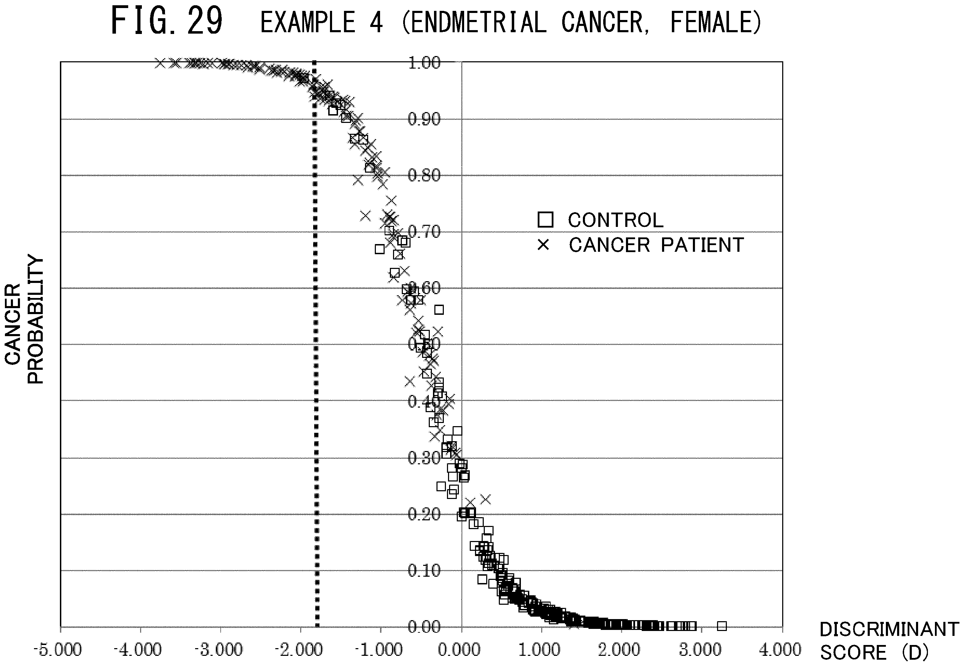

Using these results, similar to the example 1, the discriminant score was calculated by inputting the concentration data of the 17 trace elements (the set of evaluation elements) contained in the serums and the age data of the subjects into the aforementioned discriminant and then, the risk (probability) of suffering from endometrial cancer was calculated using the discriminant score thus calculated. The result of this calculation is shown in Fig. 29.

As seen from Fig. 29, in the case of female endometrial cancer, as the discriminant score having a negative value increases, the risk rises. Specifically, it can be interpreted that acquiring endometrial cancer is estimated with a probability of 95% or higher when the value of the discriminant score is approximately equal to -1.8 or lower.

EXAMPLE 5

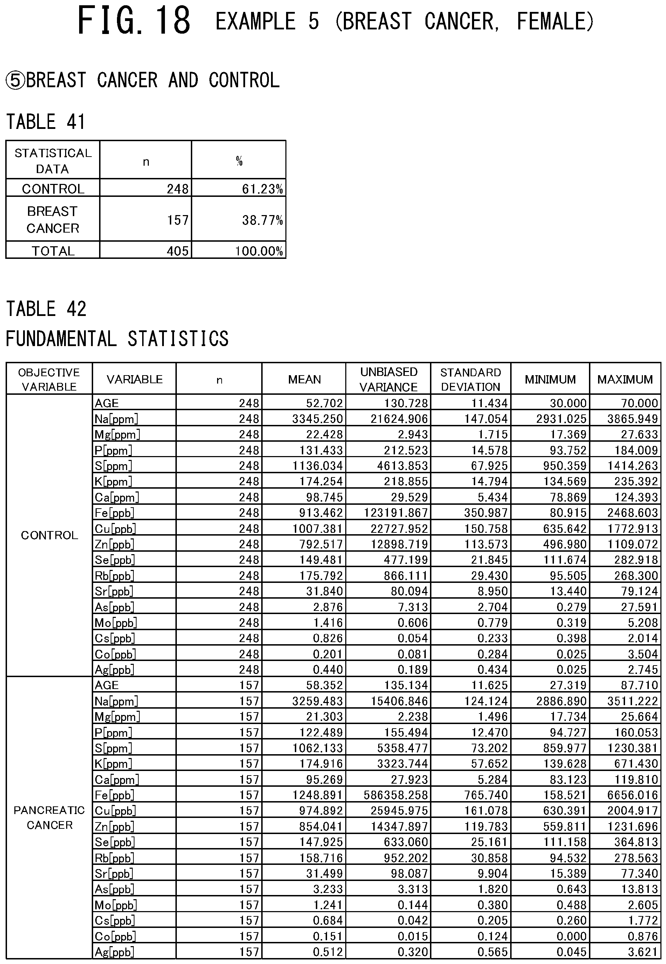

In the example 5, the risk of suffering from female breast cancer was estimated. The subjects whose cancer risk was to be estimated in this example were 157 subjects who belonged to the case group (female breast cancer patients), and 248 subjects who belonged to the female control group (controls) which was the same as the example 4, as shown in the table 41 of Fig. 18. The serums of these subjects were used as the evaluation targets. The data used in this evaluation were the age data of the subjects and the concentration data of the 17 elements which were used as the set of evaluation elements in the example 1.

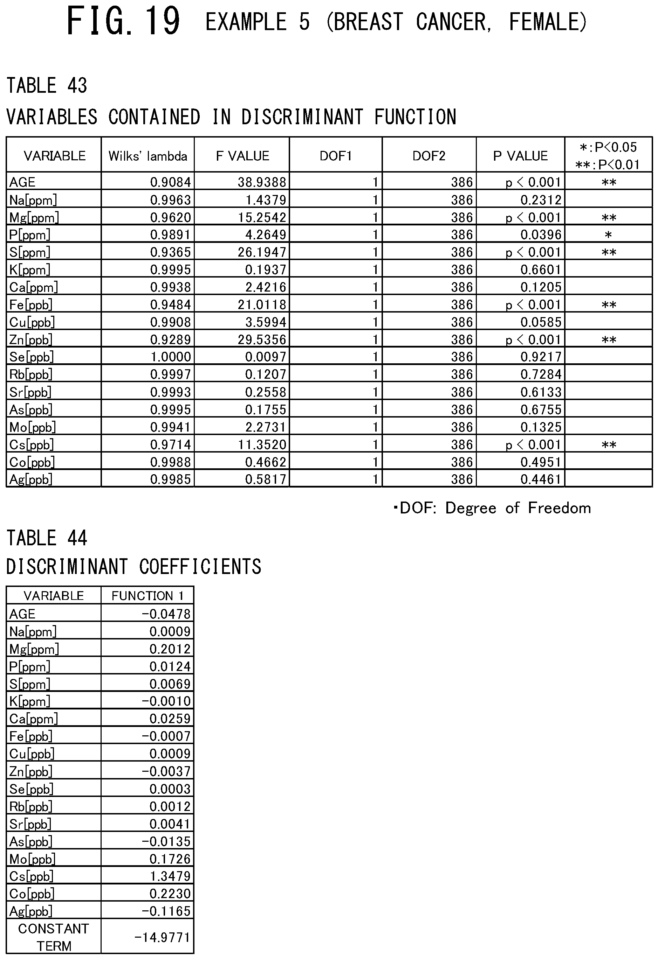

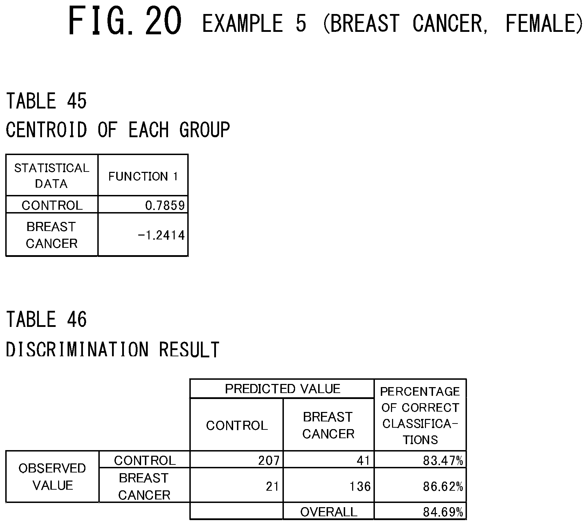

The mean value, the standard deviation, the maximum value, and the minimum value of each of the items are shown in the table 42 of Fig. 18, in which the subjects are classified into the case group (breast cancer patients) and the control group (controls). As a result of discriminant analysis, as shown in the table 43 of Fig. 19, it was found that the age data of the subjects and the 6 elements of Mg, P, S, Fe, Zn, and Cs were judged significant for discrimination. The discriminant coefficients and the constant term of the discriminant used in this discriminant analysis were shown in the Table 44 of Fig. 19. As shown in the table 45 of Fig. 20, it can be concluded that the element whose discriminant coefficient has a plus (+) sign is strongly relevant to the control group (controls) and the element whose discriminant coefficient has a minus (-) sign is strongly relevant to the case group (having breast cancer) from the centroid values of the case group (female breast cancer patients) and the control group (controls).

The discrimination result is shown in the table 46 of Fig. 20. According to this result, the 136 cases out of the 157 cases belonging to the case group were correctly classified (Sensitivity: 86.6%), and the 207 samples out of the 248 samples belonging to the control group were correctly classified (Specificity: 83.5%). Accordingly, it was indicated that the accuracy rate had a high value of 84.7%.

When calculating the Area Under the Curve (AUC) from the ROC curve which was obtained from the discriminant analysis, a high value of 0.932 was obtained, as shown in Fig, 25.

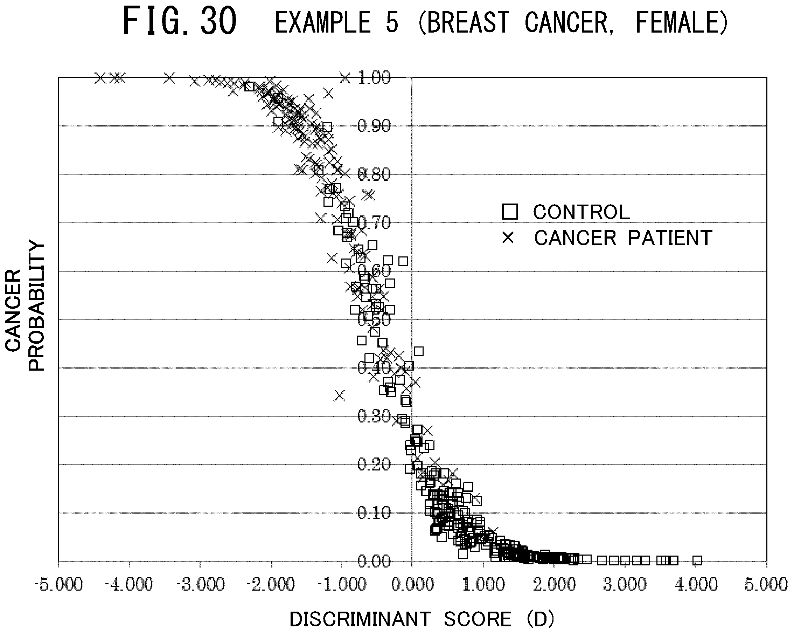

Using these results, similar to the example 1, the discriminant score was calculated by inputting the concentration data of the 17 trace elements (the set of evaluation elements) contained in the serums and the age data of the subjects into the aforementioned discriminant and then, the risk (probability) of suffering from breast cancer was calculated using the discriminant score thus calculated. The result of this calculation is shown in Fig. 30.

As seen from Fig. 30, in the case of female breast cancer, as the discriminant score having a negative value increases, the risk rises. Specifically, it can be interpreted that acquiring breast cancer is estimated with a probability of 95% or higher when the value of the discriminant score is approximately equal to -1.9 or lower.

EXAMPLE 6

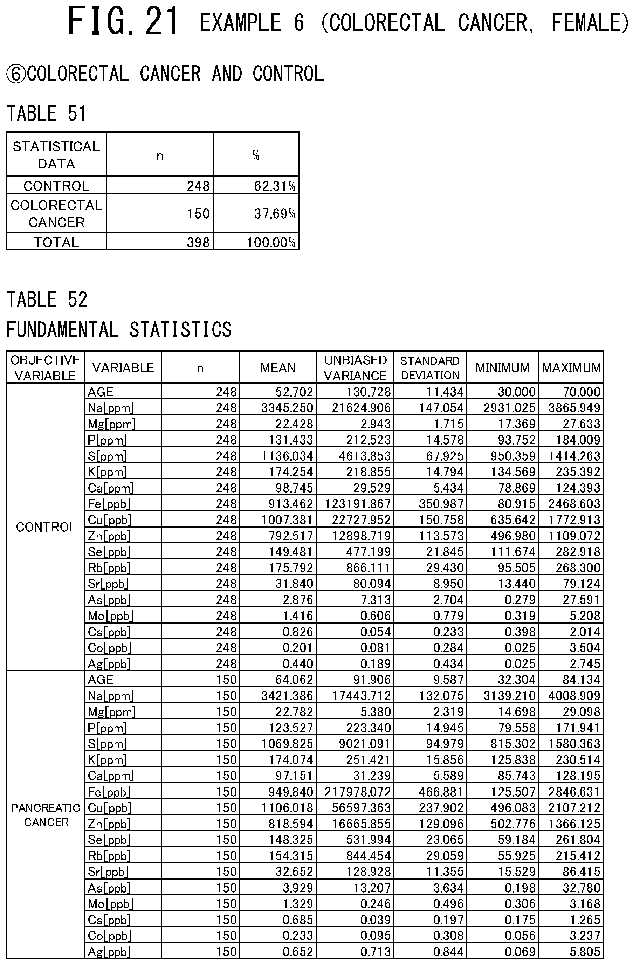

In the example 6, the risk of suffering from female colorectal cancer was estimated. The subjects whose cancer risk was to be estimated in this example were 150 subjects who belonged to the case group (female colorectal cancer patients), and 248 subjects who belonged to the female control group (controls) which was the same as the example 1, as shown in the table 51 of Fig. 21. The serums of these subjects were used as the evaluation targets. The data used in this evaluation were the age data of the subjects and the concentration data of the 17 elements which were used as the set of evaluation elements in the example 1.

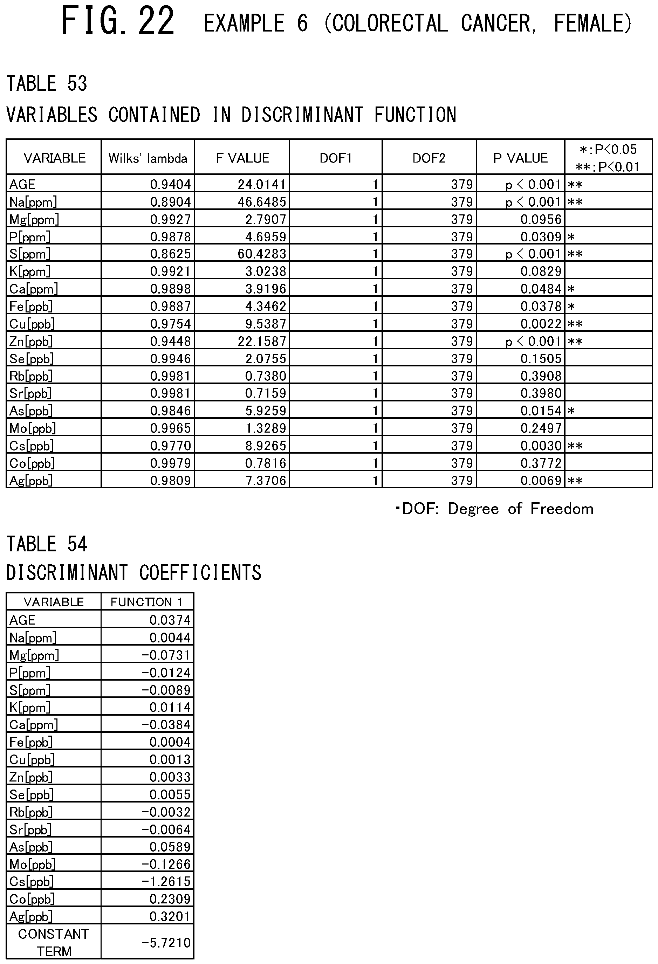

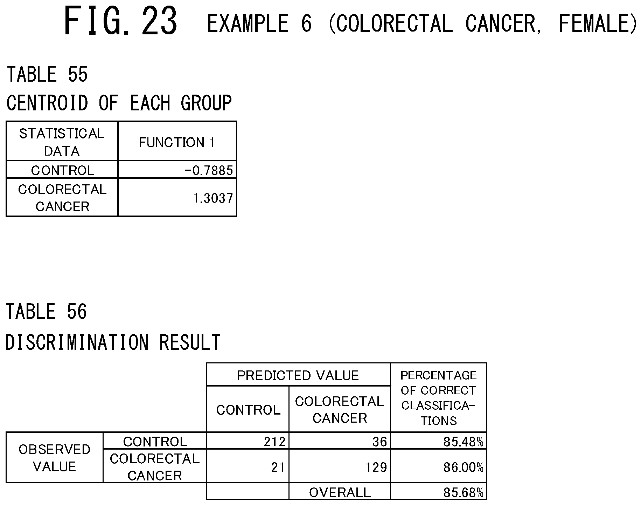

The mean value, the standard deviation, the maximum value, and the minimum value of each of the items are shown in the table 52 of Fig. 21, in which the subjects are classified into the case group (colorectal cancer patients) and the control group (controls). As a result of discriminant analysis, as shown in the table 53 of Fig. 22, it was found that the age data of the subjects and the 10 elements of Na, P, S, Ca, Fe, Cu, Zn, As, Cs, and Ag were judged significant for discrimination. The discriminant coefficients and the constant term of the discriminant used in this discriminant analysis were shown in the Table 54 of Fig. 22. As shown in the table 55 of Fig. 23, it can be concluded that the element whose discriminant coefficient has as plus (+) sign is strongly relevant to the case group (having colorectal cancer) and the element whose discriminant coefficient has a minus (-) sign is strongly relevant to the control group from the centroid values of the case group (female colorectal cancer patients) and the control group (controls).

The discrimination result is shown in the table 56 of Fig. 23. According to this result, the 129 cases out of the 150 cases belonging to the case group were correctly classified (Sensitivity: 86.0%), and the 212 samples out of the 248 samples belonging to the control group were correctly classified (Specificity: 85.5%). Accordingly, it was indicated that the accuracy rate had a high value of 85.7%.

When calculating the Area Under the Curve (AUC) from the ROC curve which was obtained from the discriminant analysis, a high value of 0.930 was obtained, as shown in Fig, 25.

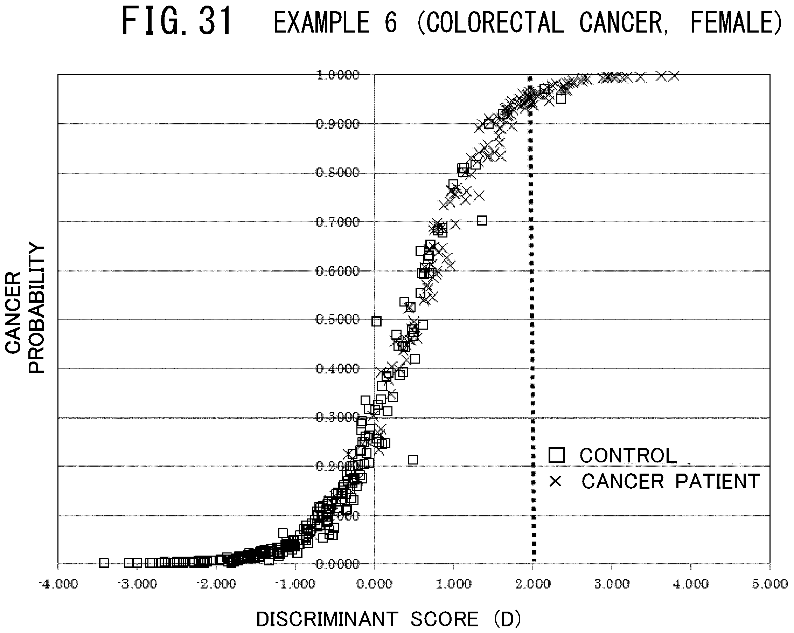

Using these results, similar to the example 1, the discriminant score was calculated by inputting the concentration data of the 17 trace elements (the set of evaluation elements) contained in the serums and the age data of the subjects into the aforementioned discriminant and then, the risk (probability) of suffering from colorectal cancer was calculated using the discriminant score thus calculated. The result of this calculation is shown in Fig. 31.

As seen from Fig. 31, in the case of female colorectal cancer, as the discriminant score having a positive value increases, the risk rises. Specifically, it can be interpreted that acquiring breast cancer is estimated with a probability of 95% or higher when the value of the discriminant score is approximately equal to 2.0 or higher.

INDUSTRIAL APPLICABILITY

The present invention is widely applicable to the fields where quick and convenient estimation of the presence or absence of suffering cancer of humans (or animals) is expected.

DESCRIPTION OF REFERENCE SIGNS

1 test tube
2 serum sample
5 in-serum element concentration measurement section
10 cancer evaluation system
11 data storage section
12 discriminant function generation section
13 evaluation result operation section

## Claims

**1.** A cancer risk evaluation method comprising:

the correlation operating step of operating a correlation among concentrations of a set of evaluation elements contained in a serum which is taken from a subject by applying concentration data of the set of evaluation elements and age data of the subject to a discriminant function or functions for discriminating which of a case group and a control group the subject belongs to; and
the indicator obtaining step of obtaining an indicator for discriminating whether or not the subject suffers from any type of cancer based on the correlation operated in the correlation operating step;
wherein in the correlation operating step, a combination of 17 elements of Na, Mg, P, S, K, Ca, Fe, Cu, Zn, Se, Rb, Sr, As, Mo, Cs, Co, and Ag is used as the set of evaluation elements; and
in the indicator obtaining step, the indicator is generated based on a discriminant score or scores calculated by applying the concentration data and the age data to the discriminant function or functions which is/are used in the correlation operating step.

**2.** The cancer risk evaluation method according to claim 1, wherein in a case where the age data and the concentration data of the 9 elements of Na, P, S, Ca, Fe, Cu, Zn, Se, and Rb which are selected from the 17 elements used as the set of evaluation elements are judged significant for discrimination based on the correlation which is operated in the correlation operating step, and the subject is male, an estimate that a type of cancer of the subject is pancreatic cancer is included into the indicator.

**3.** The cancer risk evaluation method according to claim 1, wherein in a case where the age data and the concentration data of the 10 elements of Na, P, S, K, Ca, Fe, As, Sr, Rb, and Mo which are selected from the 17 elements used as the set of evaluation elements are judged significant for discrimination based on the correlation which is operated in the correlation operating step, and the subject is male, an estimate that a type of cancer of the subject is prostate cancer is included into the indicator.

**4.** The cancer risk evaluation method according to claim 1, wherein in a case where the age data and the concentration data of the 10 elements of Na, P, S, K, Cu, Zn, Rb, Se, Mo, and Co which are selected from the 17 elements used as the set of evaluation elements are judged significant for discrimination based on the correlation which is operated in the correlation operating step, and the subject is male, an estimate that a type of cancer of the subject is colorectal cancer is included into the indicator.

**5.** The cancer risk evaluation method according to claim 1, wherein in a case where the age data and the concentration data of the 6 elements of Mg, S, K, Ca, Fe, and Mo which are selected from the 17 elements used as the set of evaluation elements are judged significant for discrimination based on the correlation which is operated in the correlation operating step, and the subject is female, an estimate that a type of cancer of the subject is endometrial cancer is included into the indicator.

6. The cancer risk evaluation method according to claim 1, wherein in a case where the age data and the concentration data of the 6 elements of Mg, P, S, Fe, Zn, and Cs which are selected from the 17 elements used as the set of evaluation elements are judged significant for discrimination based on the correlation which is operated in the correlation operating step, and the subject is female, an estimate that a type of cancer of the subject is breast cancer is included into the indicator.

7. The cancer risk evaluation method according to claim 1, wherein in a case where the age data and the concentration data of the 10 elements of Na, P, S, Ca, Fe, Cu, Zn, As, Cs, and Ag which are selected from the 17 elements used as the set of evaluation elements are judged significant for discrimination based on the correlation which is operated in the correlation operating step, and the subject is female, an estimate that a type of cancer of the subject is colorectal cancer is included into the indicator.

8. A cancer risk evaluation system comprising:

a data storage section for storing concentration data of a set of evaluation elements contained in a blood which is taken from a subject and age data of the subject;
a discriminant function generation section for generating a discriminant function or functions for discriminating which of a case group and a control group the subject belongs to; and
an evaluation result operation section for operating a correlation among concentrations of the set of evaluation elements contained in the serum by applying the concentration data of the subject and the age data thereof stored in the data storage section to a discriminant function or functions generated by the discriminant function generation section, thereby outputting an evaluation result that discriminates whether or not the subject suffers from any type of cancer based on the correlation;
wherein a combination of 17 elements of Na, Mg, P, S, K, Ca, Fe, Cu, Zn, Se, Rb, Sr, As, Mo, Cs, Co, and Ag is used as the set of evaluation elements; and
in the evaluation result operation section, a discriminant score or scores is/are calculated by applying the concentration data and the age data which are stored in the data storage section to the discriminant function or functions which is/are generated by the discriminant function generation section, and the evaluation result is generated based on the discriminant scores.

9. The cancer risk evaluation system according to claim 8, wherein in a case where the age data and the concentration data of the 9 elements of Na, P, S, Ca, Fe, Cu, Zn, Se, and Rb which are selected from the 17 elements used as the set of evaluation elements are judged significant for discrimination based on the correlation which is operated by the evaluation result operation section, and the subject is male, an estimate that a type of cancer of the subject is pancreatic cancer is included into the evaluation result.

10. The cancer risk evaluation system according to claim 8, wherein in a case where the age data and the concentration data of the 10 elements of Na, P, S, K, Ca, Fe, As, Sr, Rb, and Mo which are selected from the 17 elements used as the set of evaluation elements are judged significant for discrimination based on the correlation which is operated by the evaluation result operation section, and the subject is male, an estimate that a type of cancer of the subject is prostate cancer is included into the evaluation result.

11. The cancer risk evaluation system according to claim 8, wherein in a case where the age data and the concentration data of the 10 elements of Na, P, S, K, Cu, Zn, Rb, Se, Mo, and Co which are selected from the 17 elements used as the set of evaluation elements are judged significant for discrimination based on the correlation which is operated by the evaluation result operation section, and the subject is male, an estimate that a type of cancer of the subject is colorectal cancer is included into the evaluation result.

12. The cancer risk evaluation system according to claim 8, wherein in a case where the age data and the concentration data of the 6 elements of Mg, S, K, Ca, Fe, and Mo which are selected from the 17 elements used as the set of evaluation elements are judged significant for discrimination based on the correlation which is operated by the evaluation result operation section, and the subject is female, an estimate that a type of cancer of the subject is endometrial cancer is included into the evaluation result.

13. The cancer risk evaluation system according to claim 8, wherein in a case where the age data and the concentration data of the 6 elements of Mg, P, S, Fe, Zn, and Cs which are selected from the 17 elements used as the set of evaluation elements are judged significant for discrimination based on the correlation which is operated by the evaluation result operation section, and the subject is female, an estimate that a type of cancer of the subject is breast cancer is included into the evaluation result.

**14.** The cancer risk evaluation system according to claim 8, wherein in a case where the age data and the concentration data of the 10 elements of Na, P, S, Ca, Fe, Cu, Zn, As, Cs, and Ag which are selected from the 17 elements used as the set of evaluation elements are judged significant for discrimination based on the correlation which is operated by the evaluation result operation section, and the subject is female, an estimate that a type of cancer of the subject is colorectal cancer is included into the evaluation result.

FIG. 1

BASIC PRINCIPLE OF
CANCER RISK EVALUATION METHOD OF INVENTION

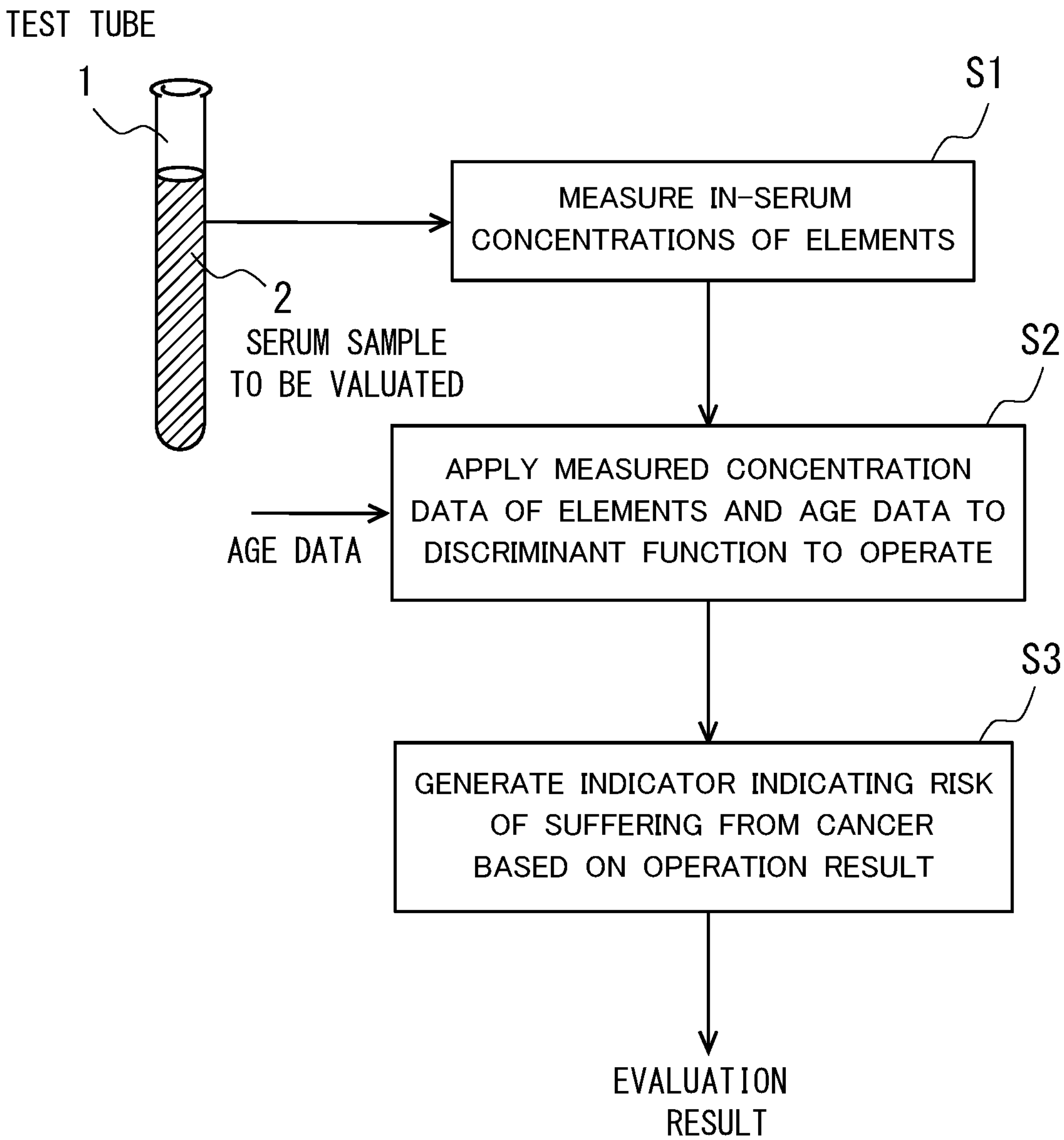

FIG. 2

BASIC STRUCTURE OF
CANCER RISK EVALUATION SYSTEM OF INVENTION

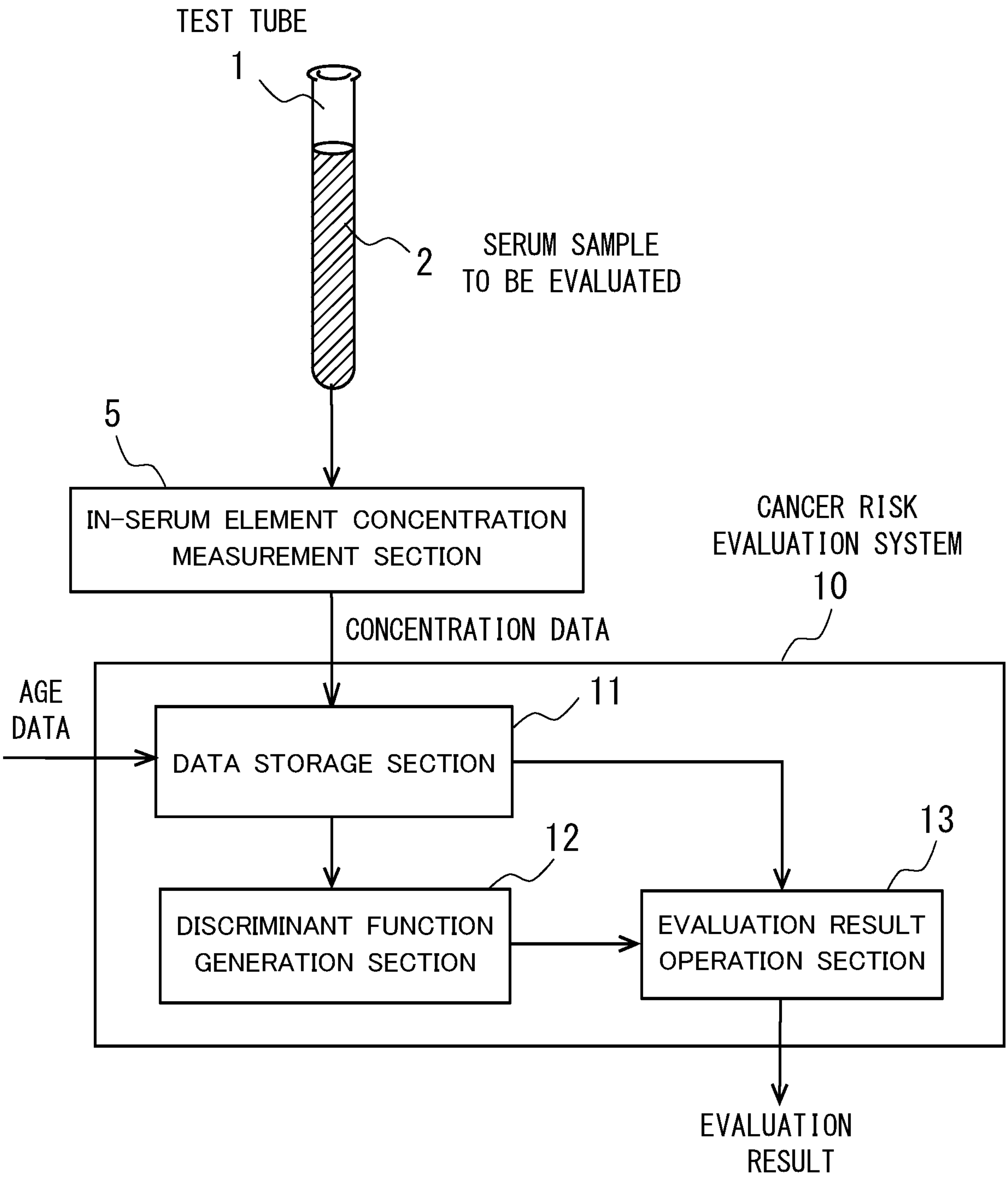

# FIG. 3

| AGE CLASS | MALE | | FEMALE | | TOTAL | |
|---|---|---|---|---|---|---|
| | NUMBER | % | NUMBER | % | NUMBER | % |
| 20-29 | 0 | - | 1 | 0.2 | 1 | 0.1 |
| 30-39 | 8 | 1.9 | 13 | 2.8 | 21 | 2.4 |
| 40-49 | 30 | 7.3 | 65 | 14.1 | 95 | 10.9 |
| 50-59 | 72 | 17.5 | 132 | 28.6 | 204 | 23.3 |
| 60-69 | 184 | 44.7 | 150 | 32.5 | 334 | 38.2 |
| 70-79 | 107 | 26.0 | 92 | 19.9 | 199 | 22.8 |
| 80-89 | 11 | 2.7 | 9 | 1.9 | 20 | 2.3 |
| TOTAL | 412 | 100.0 | 462 | 100.0 | 874 | 100.0 |

# FIG. 4

| SEX | CANCER PATIENT | | CONTROL |
|---|---|---|---|
| | SITE | NUMBER | NUMBER |
| MALE | PANCREATIC CANCER | 144 | 364 |
| | PROSTATE CANCER | 94 | |
| | COLORECTAL CANCER | 174 | |
| FEMALE | ENDOMETRIAL CANCER | 155 | 248 |
| | BREAST CANCER | 157 | |
| | COLORECTAL CANCER | 150 | |

FIG. 5

| ITEM | MALE | | | FEMALE | | |
|---|---|---|---|---|---|---|
| | PANCREATIC CANCER | PROSTATE CANCER | COLORECTAL CANCER | BREAST CANCER | ENDOMETRIAL CANCER | COLORECTAL CANCER |
| AGE | + | ++ | + | ++ | ++ | ++ |
| Na(ppm) | -- | ++ | ++ | | | ++ |
| K(ppm) | | ++ | + | | -- | |
| Rb(ppb) | - | -- | -- | | | |
| Cs(ppb) | | | | | | -- |
| Mg(ppm) | | | | -- | -- | |
| Ca(ppm) | ++ | -- | | | + | - |
| Sr(ppb) | | -- | | | | |
| P(ppm) | - | - | -- | - | | - |
| As(ppb) | | ++ | | ++ | | + |
| S(ppm) | -- | -- | -- | -- | -- | -- |
| Se(ppb) | -- | | ++ | | | |
| Fe(ppb) | - | ++ | | ++ | + | + |
| Co(ppb) | | | ++ | | | |
| Cu(ppb) | ++ | | ++ | | | ++ |
| Zn(ppb) | ++ | | ++ | ++ | | ++ |
| Mo(ppb) | | -- | -- | | ++ | |
| Ag(ppb) | | | | | | ++ |

-/+:P<0.05, --/++:P<0.01

# FIG. 6 EXAMPLE 1 (PANCREATIC CANCER, MALE)

## ①PANCREATIC CANCER AND CONTROL

TABLE 1

| STATISTICAL DATA | n | % |
|---|---|---|
| CONTROL | 364 | 71.65% |
| PANCREATIC CANCER | 144 | 28.35% |
| TOTAL | 508 | 100.00% |

TABLE 2

FUNDAMENTAL STATISTICS

| OBJECTIVE VARIABLE | VARIABLE | n | MEAN | UNBIASED VARIANCE | STANDARD DEVIATION | MINIMUM | MAXIMUM |
|---|---|---|---|---|---|---|---|
| CONTROL | AGE | 364 | 61.360 | 100.281 | 10.014 | 30.000 | 75.000 |
| | Na[ppm] | 364 | 3344.564 | 18955.992 | 137.681 | 3080.800 | 3923.920 |
| | Mg[ppm] | 364 | 21.779 | 3.105 | 1.762 | 16.335 | 27.331 |
| | P[ppm] | 364 | 124.503 | 227.310 | 15.077 | 89.141 | 174.475 |
| | S[ppm] | 364 | 1173.032 | 6899.872 | 83.065 | 958.591 | 1579.547 |
| | K[ppm] | 364 | 177.355 | 255.223 | 15.976 | 129.798 | 258.005 |
| | Ca[ppm] | 364 | 98.214 | 36.603 | 6.050 | 78.310 | 124.278 |
| | Fe[ppb] | 364 | 1072.490 | 117801.352 | 343.222 | 288.039 | 2664.570 |
| | Cu[ppb] | 364 | 907.634 | 22419.414 | 149.731 | 551.814 | 1567.016 |
| | Zn[ppb] | 364 | 776.736 | 14247.297 | 119.362 | 539.554 | 1344.875 |
| | As[ppb] | 364 | 3.548 | 6.419 | 2.533 | 0.351 | 17.053 |
| | Sr[ppb] | 364 | 35.049 | 146.967 | 12.123 | 15.112 | 101.955 |
| | Rb[ppb] | 364 | 177.180 | 1138.239 | 33.738 | 90.736 | 328.403 |
| | Se[ppb] | 364 | 151.910 | 396.427 | 19.910 | 107.837 | 277.093 |
| | Mo[ppb] | 364 | 1.753 | 1.592 | 1.262 | 0.371 | 15.379 |
| | Cs[ppb] | 364 | 0.793 | 0.056 | 0.237 | 0.331 | 1.882 |
| | Co[ppb] | 364 | 0.109 | 0.006 | 0.081 | 0.046 | 0.893 |
| | Ag[ppb] | 364 | 0.367 | 0.257 | 0.507 | 0.007 | 5.983 |
| PANCREATIC CANCER | AGE | 144 | 64.243 | 84.591 | 9.197 | 31.000 | 85.000 |
| | Na[ppm] | 144 | 3225.531 | 10763.646 | 103.748 | 2666.295 | 3562.935 |
| | Mg[ppm] | 144 | 20.596 | 4.714 | 2.171 | 15.062 | 29.805 |
| | P[ppm] | 144 | 114.424 | 447.349 | 21.151 | 73.230 | 256.699 |
| | S[ppm] | 144 | 1089.444 | 9609.034 | 98.026 | 816.078 | 1338.973 |
| | K[ppm] | 144 | 171.626 | 280.727 | 16.755 | 132.142 | 240.121 |
| | Ca[ppm] | 144 | 95.474 | 29.909 | 5.469 | 79.108 | 120.140 |
| | Fe[ppb] | 144 | 823.291 | 156864.602 | 396.061 | 210.313 | 2524.643 |
| | Cu[ppb] | 144 | 1184.775 | 138109.281 | 371.631 | 488.782 | 2848.773 |
| | Zn[ppb] | 144 | 765.204 | 23196.036 | 152.302 | 449.337 | 1386.572 |
| | As[ppb] | 144 | 2.867 | 5.716 | 2.391 | 0.261 | 15.859 |
| | Sr[ppb] | 144 | 32.918 | 138.612 | 11.773 | 13.275 | 97.864 |
| | Rb[ppb] | 144 | 155.970 | 900.935 | 30.016 | 99.683 | 270.986 |
| | Se[ppb] | 144 | 133.524 | 369.937 | 19.234 | 73.274 | 212.776 |
| | Mo[ppb] | 144 | 1.883 | 1.076 | 1.037 | 0.425 | 7.229 |
| | Cs[ppb] | 144 | 0.657 | 0.030 | 0.174 | 0.243 | 1.085 |
| | Co[ppb] | 144 | 0.128 | 0.005 | 0.068 | 0.055 | 0.480 |
| | Ag[ppb] | 144 | 0.394 | 0.191 | 0.437 | 0.011 | 2.653 |

# FIG. 7 EXAMPLE 1 (PANCREATIC CANCER, MALE)

## TABLE 3
## VARIABLES CONTAINED IN DISCRIMINANT FUNCTION

| VARIABLE | Wilks' lambda | F VALUE | DOF1 | DOF2 | P VALUE | *: P<0.05<br>**: P<0.01 |
|---|---|---|---|---|---|---|
| AGE | 0.9870 | 6.4309 | 1 | 489 | 0.0115 | * |
| Na[ppm] | 0.9650 | 17.7170 | 1 | 489 | p < 0.001 | ** |
| Mg[ppm] | 1.0000 | 0.0141 | 1 | 489 | 0.9056 | |
| P[ppm] | 0.9896 | 5.1262 | 1 | 489 | 0.0240 | * |
| S[ppm] | 0.9667 | 16.8377 | 1 | 489 | p < 0.001 | ** |
| K[ppm] | 1.0000 | 0.0226 | 1 | 489 | 0.8804 | |
| Ca[ppm] | 0.9711 | 14.5680 | 1 | 489 | p < 0.001 | ** |
| Fe[ppb] | 0.9900 | 4.9283 | 1 | 489 | 0.0269 | * |
| Cu[ppb] | 0.8777 | 68.1614 | 1 | 489 | p < 0.001 | ** |
| Zn[ppb] | 0.9792 | 10.3941 | 1 | 489 | 0.0013 | ** |
| As[ppb] | 0.9992 | 0.3729 | 1 | 489 | 0.5417 | |
| Sr[ppb] | 0.9986 | 0.7059 | 1 | 489 | 0.4012 | |
| Rb[ppb] | 0.9874 | 6.2323 | 1 | 489 | 0.0129 | * |
| Se[ppb] | 0.9826 | 8.6541 | 1 | 489 | 0.0034 | ** |
| Mo[ppb] | 0.9992 | 0.3793 | 1 | 489 | 0.5383 | |
| Cs[ppb] | 0.9925 | 3.7012 | 1 | 489 | 0.0550 | |
| Co[ppb] | 1.0000 | 0.0224 | 1 | 489 | 0.8811 | |
| Ag[ppb] | 1.0000 | 0.0078 | 1 | 489 | 0.9298 | |

・DOF: Degree of Freedom

## TABLE 4
## DISCRIMINANT COEFFICIENTS

| VARIABLE | FUNCTION 1 |
|---|---|
| AGE | -0.0188 |
| Na[ppm] | 0.0031 |
| Mg[ppm] | 0.0052 |
| P[ppm] | 0.0101 |
| S[ppm] | 0.0048 |
| K[ppm] | -0.0009 |
| Ca[ppm] | -0.0680 |
| Fe[ppb] | 0.0005 |
| Cu[ppb] | -0.0024 |
| Zn[ppb] | -0.0020 |
| As[ppb] | 0.0179 |
| Sr[ppb] | 0.0050 |
| Rb[ppb] | 0.0082 |
| Se[ppb] | 0.0119 |
| Mo[ppb] | 0.0378 |
| Cs[ppb] | 0.7887 |
| Co[ppb] | -0.1346 |
| Ag[ppb] | -0.0126 |
| CONSTANT TERM | -9.7483 |

# FIG. 8 EXAMPLE 1 (PANCREATIC CANCER, MALE)

TABLE 5

CENTROID OF EACH GROUP

| STATISTICAL DATA | FUNCTION 1 |
|---|---|
| CONTROL | 0.5683 |
| PANCREATIC CANCER | −1.4367 |

TABLE 6

DISCRIMINATION RESULT

| | | PREDICTED VALUE | | PERCENTAGE OF CORRECT CLASSIFICA-TIONS |
|---|---|---|---|---|
| | | CONTROL | PANCREATIC CANCER | |
| OBSERVED VALUE | CONTROL | 329 | 35 | 90.38% |
| | PANCREATIC CANCER | 31 | 113 | 78.47% |
| | | | OVERALL | 87.01% |

# FIG. 9 EXAMPLE 2 (PROSTATE CANCER, MALE)

## ②PROSTATE CANCER AND CONTROL

### TABLE 11

| STATISTICAL DATA | n | % |
|---|---|---|
| CONTROL | 364 | 79.48% |
| PROSTATE CANCER | 94 | 20.52% |
| TOTAL | 458 | 100.00% |

### TABLE 12
### FUNDAMENTAL STATISTICS

| OBJECTIVE VARIABLE | VARIABLE | n | MEAN | UNBIASED VARIANCE | STANDARD DEVIATION | MINIMUM | MAXIMUM |
|---|---|---|---|---|---|---|---|
| CONTROL | AGE | 364 | 61.360 | 100.281 | 10.014 | 30.000 | 75.000 |
| | Na[ppm] | 364 | 3344.564 | 18955.992 | 137.681 | 3080.800 | 3923.920 |
| | Mg[ppm] | 364 | 21.779 | 3.105 | 1.762 | 16.335 | 27.331 |
| | P[ppm] | 364 | 124.503 | 227.310 | 15.077 | 89.141 | 174.475 |
| | S[ppm] | 364 | 1173.032 | 6899.872 | 83.065 | 958.591 | 1579.547 |
| | K[ppm] | 364 | 177.355 | 255.223 | 15.976 | 129.798 | 258.005 |
| | Ca[ppm] | 364 | 98.214 | 36.603 | 6.050 | 78.310 | 124.278 |
| | Fe[ppb] | 364 | 1072.490 | 117801.352 | 343.222 | 288.039 | 2664.570 |
| | Cu[ppb] | 364 | 907.634 | 22419.414 | 149.731 | 551.814 | 1567.016 |
| | Zn[ppb] | 364 | 776.736 | 14247.297 | 119.362 | 539.554 | 1344.875 |
| | As[ppb] | 364 | 3.548 | 6.419 | 2.533 | 0.351 | 17.053 |
| | Sr[ppb] | 364 | 35.049 | 146.967 | 12.123 | 15.112 | 101.955 |
| | Rb[ppb] | 364 | 177.180 | 1138.239 | 33.738 | 90.736 | 328.403 |
| | Se[ppb] | 364 | 151.910 | 396.427 | 19.910 | 107.837 | 277.093 |
| | Mo[ppb] | 364 | 1.753 | 1.592 | 1.262 | 0.371 | 15.379 |
| | Cs[ppb] | 364 | 0.793 | 0.056 | 0.237 | 0.331 | 1.882 |
| | Co[ppb] | 364 | 0.109 | 0.006 | 0.081 | 0.046 | 0.893 |
| | Ag[ppb] | 364 | 0.367 | 0.257 | 0.507 | 0.007 | 5.983 |
| PANCREATIC CANCER | AGE | 94 | 105.616 | 11999.988 | 109.544 | 39.420 | 468.000 |
| | Na[ppm] | 94 | 3321.671 | 24571.776 | 156.754 | 2780.868 | 3741.222 |
| | Mg[ppm] | 94 | 21.083 | 3.543 | 1.882 | 15.051 | 25.900 |
| | P[ppm] | 94 | 111.717 | 223.902 | 14.963 | 86.992 | 169.307 |
| | S[ppm] | 94 | 1046.699 | 6740.148 | 82.098 | 803.718 | 1264.607 |
| | K[ppm] | 94 | 177.036 | 653.366 | 25.561 | 122.828 | 231.518 |
| | Ca[ppm] | 94 | 92.604 | 93.371 | 9.663 | 71.431 | 108.346 |
| | Fe[ppb] | 94 | 1264.992 | 237661.111 | 487.505 | 454.327 | 3060.553 |
| | Cu[ppb] | 94 | 865.259 | 33335.359 | 182.580 | 529.146 | 1738.518 |
| | Zn[ppb] | 94 | 713.266 | 11713.833 | 108.230 | 420.396 | 1006.582 |
| | As[ppb] | 94 | 4.198 | 15.311 | 3.913 | 0.936 | 33.124 |
| | Sr[ppb] | 94 | 30.977 | 89.655 | 9.469 | 16.933 | 60.791 |
| | Rb[ppb] | 94 | 161.896 | 1114.683 | 33.387 | 88.042 | 263.321 |
| | Se[ppb] | 94 | 142.768 | 407.743 | 20.193 | 99.067 | 210.954 |
| | Mo[ppb] | 94 | 1.514 | 0.521 | 0.722 | 0.442 | 4.669 |
| | Cs[ppb] | 94 | 0.695 | 0.034 | 0.183 | 0.313 | 1.236 |
| | Co[ppb] | 94 | 0.110 | 0.003 | 0.052 | 0.047 | 0.338 |
| | Ag[ppb] | 94 | 0.584 | 3.328 | 1.824 | 0.028 | 16.967 |

# FIG. 10 EXAMPLE 2 (PROSTATE CANCER, MALE)

TABLE 13

VARIABLES CONTAINED IN DISCRIMINANT FUNCTION

| VARIABLE | Wilks' lambda | F VALUE | DOF1 | DOF2 | P VALUE | *: P<0.05 **: P<0.01 |
|---|---|---|---|---|---|---|
| AGE | 0.9561 | 20.1552 | 1 | 439 | p < 0.001 | ** |
| Na[ppm] | 0.9409 | 27.5582 | 1 | 439 | p < 0.001 | ** |
| Mg[ppm] | 1.0000 | 0.0162 | 1 | 439 | 0.8986 | |
| P[ppm] | 0.9864 | 6.0346 | 1 | 439 | 0.0144 | * |
| S[ppm] | 0.8591 | 71.9999 | 1 | 439 | p < 0.001 | ** |
| K[ppm] | 0.9614 | 17.6430 | 1 | 439 | p < 0.001 | ** |
| Ca[ppm] | 0.9754 | 11.0877 | 1 | 439 | p < 0.001 | ** |
| Fe[ppb] | 0.9374 | 29.3051 | 1 | 439 | p < 0.001 | ** |
| Cu[ppb] | 0.9998 | 0.1028 | 1 | 439 | 0.7486 | |
| Zn[ppb] | 0.9996 | 0.1972 | 1 | 439 | 0.6572 | |
| As[ppb] | 0.9842 | 7.0606 | 1 | 439 | 0.0082 | ** |
| Sr[ppb] | 0.9814 | 8.3055 | 1 | 439 | 0.0041 | ** |
| Rb[ppb] | 0.9768 | 10.4445 | 1 | 439 | 0.0013 | ** |
| Se[ppb] | 0.9939 | 2.6925 | 1 | 439 | 0.1015 | |
| Mo[ppb] | 0.9721 | 12.5853 | 1 | 439 | p < 0.001 | ** |
| Cs[ppb] | 0.9999 | 0.0638 | 1 | 439 | 0.8007 | |
| Co[ppb] | 0.9949 | 2.2343 | 1 | 439 | 0.1357 | |
| Ag[ppb] | 0.9953 | 2.0753 | 1 | 439 | 0.1504 | |

・DOF: Degree of Freedom

TABLE 14

DISCRIMINANT COEFFICIENTS

| VARIABLE | FUNCTION 1 |
|---|---|
| AGE | 0.0061 |
| Na[ppm] | 0.0037 |
| Mg[ppm] | 0.0061 |
| P[ppm] | −0.0128 |
| S[ppm] | −0.0102 |
| K[ppm] | 0.0239 |
| Ca[ppm] | −0.0580 |
| Fe[ppb] | 0.0010 |
| Cu[ppb] | 0.0002 |
| Zn[ppb] | 0.0003 |
| As[ppb] | 0.0678 |
| Sr[ppb] | −0.0177 |
| Rb[ppb] | −0.0104 |
| Se[ppb] | 0.0066 |
| Mo[ppb] | −0.2240 |
| Cs[ppb] | −0.1026 |
| Co[ppb] | 1.3779 |
| Ag[ppb] | 0.1092 |
| CONSTANT TERM | 1.7248 |

# FIG. 11 EXAMPLE 2 (PROSTATE CANCER, MALE)

TABLE 15
CENTROID OF EACH GROUP

| STATISTICAL DATA | FUNCTION 1 |
|---|---|
| CONTROL | −0.4978 |
| PROSTATE CANCER | 1.9278 |

TABLE 16
DISCRIMINATION RESULT

| | | PREDICTED VALUE | | PERCENTAGE OF CORRECT CLASSIFICATIONS |
|---|---|---|---|---|
| | | CONTROL | PROSTATE CANCER | |
| OBSERVED VALUE | CONTROL | 330 | 34 | 90.66% |
| | PROSTATE CANCER | 13 | 81 | 86.17% |
| | | | OVERALL | 89.74% |

# FIG. 12 EXAMPLE 3 (COLORECTAL CANCER, MALE)

## ③COLORECTAL CANCER AND CONTROL

TABLE 21

| STATISTICAL DATA | n | % |
|---|---|---|
| CONTROL | 364 | 67.66% |
| COLORECTAL CANCER | 174 | 32.34% |
| TOTAL | 538 | 100.00% |

TABLE 22

FUNDAMENTAL STATISTICS

| OBJECTIVE VARIABLE | VARIABLE | n | MEAN | UNBIASED VARIANCE | STANDARD DEVIATION | MINIMUM | MAXIMUM |
|---|---|---|---|---|---|---|---|
| CONTROL | AGE | 364 | 61.360 | 100.281 | 10.014 | 30.000 | 75.000 |
| | Na[ppm] | 364 | 3344.564 | 18955.992 | 137.681 | 3080.800 | 3923.920 |
| | Mg[ppm] | 364 | 21.779 | 3.105 | 1.762 | 16.335 | 27.331 |
| | P[ppm] | 364 | 124.503 | 227.310 | 15.077 | 89.141 | 174.475 |
| | S[ppm] | 364 | 1173.032 | 6899.872 | 83.065 | 958.591 | 1579.547 |
| | K[ppm] | 364 | 177.355 | 255.223 | 15.976 | 129.798 | 258.005 |
| | Ca[ppm] | 364 | 98.214 | 36.603 | 6.050 | 78.310 | 124.278 |
| | Fe[ppb] | 364 | 1072.490 | 117801.352 | 343.222 | 288.039 | 2664.570 |
| | Cu[ppb] | 364 | 907.634 | 22419.414 | 149.731 | 551.814 | 1567.016 |
| | Zn[ppb] | 364 | 776.736 | 14247.297 | 119.362 | 539.554 | 1344.875 |
| | As[ppb] | 364 | 3.548 | 6.419 | 2.533 | 0.351 | 17.053 |
| | Sr[ppb] | 364 | 35.049 | 146.967 | 12.123 | 15.112 | 101.955 |
| | Rb[ppb] | 364 | 177.180 | 1138.239 | 33.738 | 90.736 | 328.403 |
| | Se[ppb] | 364 | 151.910 | 396.427 | 19.910 | 107.837 | 277.093 |
| | Mo[ppb] | 364 | 1.753 | 1.592 | 1.262 | 0.371 | 15.379 |
| | Cs[ppb] | 364 | 0.793 | 0.056 | 0.237 | 0.331 | 1.882 |
| | Co[ppb] | 364 | 0.109 | 0.006 | 0.081 | 0.046 | 0.893 |
| | Ag[ppb] | 364 | 0.367 | 0.257 | 0.507 | 0.007 | 5.983 |
| PANCREATIC CANCER | AGE | 174 | 63.326 | 73.027 | 8.546 | 35.404 | 87.135 |
| | Na[ppm] | 174 | 3262.573 | 36148.378 | 190.127 | 2298.466 | 4118.851 |
| | Mg[ppm] | 174 | 21.337 | 3.992 | 1.998 | 14.225 | 27.782 |
| | P[ppm] | 174 | 109.841 | 204.132 | 14.287 | 76.392 | 155.493 |
| | S[ppm] | 174 | 1025.638 | 10860.222 | 104.212 | 664.698 | 1452.943 |
| | K[ppm] | 174 | 173.695 | 366.199 | 19.136 | 115.528 | 242.765 |
| | Ca[ppm] | 174 | 91.445 | 54.020 | 7.350 | 66.936 | 116.556 |
| | Fe[ppb] | 174 | 963.550 | 324937.985 | 570.033 | 82.105 | 3488.577 |
| | Cu[ppb] | 174 | 1014.948 | 49625.398 | 222.768 | 474.098 | 1728.105 |
| | Zn[ppb] | 174 | 767.968 | 17091.710 | 130.735 | 373.563 | 1124.602 |
| | As[ppb] | 174 | 3.465 | 5.446 | 2.334 | 0.142 | 17.591 |
| | Sr[ppb] | 174 | 33.615 | 144.334 | 12.014 | 15.367 | 97.650 |
| | Rb[ppb] | 174 | 163.923 | 1335.797 | 36.549 | 75.327 | 367.643 |
| | Se[ppb] | 174 | 142.620 | 593.554 | 24.363 | 38.551 | 221.766 |
| | Mo[ppb] | 174 | 1.282 | 0.332 | 0.576 | 0.219 | 4.860 |
| | Cs[ppb] | 174 | 0.731 | 0.045 | 0.213 | 0.216 | 1.797 |
| | Co[ppb] | 174 | 0.218 | 0.038 | 0.194 | 0.049 | 1.168 |
| | Ag[ppb] | 174 | 0.449 | 0.381 | 0.617 | 0.009 | 6.054 |

# FIG. 13 EXAMPLE 3 (COLORECTAL CANCER, MALE)

TABLE 23

VARIABLES CONTAINED IN DISCRIMINANT FUNCTION

| VARIABLE | Wilks' lambda | F VALUE | DOF1 | DOF2 | P VALUE | *: P<0.05<br>**: P<0.01 |
|---|---|---|---|---|---|---|
| AGE | 0.9883 | 6.1602 | 1 | 519 | 0.0134 | * |
| Na[ppm] | 0.9645 | 19.1256 | 1 | 519 | p < 0.001 | ** |
| Mg[ppm] | 0.9986 | 0.7071 | 1 | 519 | 0.4008 | |
| P[ppm] | 0.9716 | 15.1856 | 1 | 519 | p < 0.001 | ** |
| S[ppm] | 0.7329 | 189.1852 | 1 | 519 | p < 0.001 | ** |
| K[ppm] | 0.9887 | 5.9382 | 1 | 519 | 0.0152 | * |
| Ca[ppm] | 1.0000 | 0.0123 | 1 | 519 | 0.9116 | |
| Fe[ppb] | 0.9931 | 3.6110 | 1 | 519 | 0.0580 | |
| Cu[ppb] | 0.9391 | 33.6353 | 1 | 519 | p < 0.001 | ** |
| Zn[ppb] | 0.9636 | 19.6113 | 1 | 519 | p < 0.001 | ** |
| As[ppb] | 0.9977 | 1.2157 | 1 | 519 | 0.2707 | |
| Sr[ppb] | 0.9986 | 0.7489 | 1 | 519 | 0.3872 | |
| Rb[ppb] | 0.9807 | 10.1908 | 1 | 519 | 0.0015 | ** |
| Se[ppb] | 0.9837 | 8.5967 | 1 | 519 | 0.0035 | ** |
| Mo[ppb] | 0.9696 | 16.2880 | 1 | 519 | p < 0.001 | ** |
| Cs[ppb] | 0.9995 | 0.2604 | 1 | 519 | 0.6101 | |
| Co[ppb] | 0.9372 | 34.8049 | 1 | 519 | p < 0.001 | ** |
| Ag[ppb] | 0.9999 | 0.0436 | 1 | 519 | 0.8346 | |

・DOF: Degree of Freedom

TABLE 24

DISCRIMINANT COEFFICIENTS

| VARIABLE | FUNCTION 1 |
|---|---|
| AGE | 0.0161 |
| Na[ppm] | 0.0024 |
| Mg[ppm] | 0.0317 |
| P[ppm] | -0.0176 |
| S[ppm] | -0.0129 |
| K[ppm] | 0.0122 |
| Ca[ppm] | 0.0018 |
| Fe[ppb] | 0.0003 |
| Cu[ppb] | 0.0020 |
| Zn[ppb] | 0.0026 |
| As[ppb] | 0.0276 |
| Sr[ppb] | -0.0044 |
| Rb[ppb] | -0.0085 |
| Se[ppb] | 0.0099 |
| Mo[ppb] | -0.2295 |
| Cs[ppb] | 0.1749 |
| Co[ppb] | 2.6699 |
| Ag[ppb] | -0.0227 |
| CONSTANT TERM | 0.4366 |

# FIG. 14 EXAMPLE 3 (COLORECTAL CANCER, MALE)

TABLE 25

CENTROID OF EACH GROUP

| STATISTICAL DATA | FUNCTION 1 |
|---|---|
| CONTROL | -0.8072 |
| COLORECTAL CANCER | 1.6886 |

TABLE 26

DISCRIMINATION RESULT

| | | PREDICTED VALUE | | PERCENTAGE OF CORRECT CLASSIFICA-TIONS |
|---|---|---|---|---|
| | | CONTROL | COLORECTAL CANCER | |
| OBSERVED VALUE | CONTROL | 338 | 26 | 92.86% |
| | COLORECTAL CANCER | 22 | 152 | 87.36% |
| | | | OVERALL | 91.08% |

# FIG. 15 EXAMPLE 4 (ENDOMETRIAL CANCER, FEMALE)

## ④ENDOMETRIAL CANCER AND CONTROL

### TABLE 31

| STATISTICAL DATA | n | % |
|---|---|---|
| CONTROL | 248 | 61.54% |
| ENDOMETRIAL CANCER | 155 | 38.46% |
| TOTAL | 403 | 100.00% |

### TABLE 32
### FUNDAMENTAL STATISTICS

| OBJECTIVE VARIABLE | VARIABLE | n | MEAN | UNBIASED VARIANCE | STANDARD DEVIATION | MINIMUM | MAXIMUM |
|---|---|---|---|---|---|---|---|
| CONTROL | AGE | 248 | 52.702 | 130.728 | 11.434 | 30.000 | 70.000 |
| | Na[ppm] | 248 | 3345.250 | 21624.906 | 147.054 | 2931.025 | 3865.949 |
| | Mg[ppm] | 248 | 22.428 | 2.943 | 1.715 | 17.369 | 27.633 |
| | P[ppm] | 248 | 131.433 | 212.523 | 14.578 | 93.752 | 184.009 |
| | S[ppm] | 248 | 1136.034 | 4613.853 | 67.925 | 950.359 | 1414.263 |
| | K[ppm] | 248 | 174.254 | 218.855 | 14.794 | 134.569 | 235.392 |
| | Ca[ppm] | 248 | 98.745 | 29.529 | 5.434 | 78.869 | 124.393 |
| | Fe[ppb] | 248 | 913.462 | 123191.867 | 350.987 | 80.915 | 2468.603 |
| | Cu[ppb] | 248 | 1007.381 | 22727.952 | 150.758 | 635.642 | 1772.913 |
| | Zn[ppb] | 248 | 792.517 | 12898.719 | 113.573 | 496.980 | 1109.072 |
| | Se[ppb] | 248 | 149.481 | 477.199 | 21.845 | 111.674 | 282.918 |
| | Rb[ppb] | 248 | 175.792 | 866.111 | 29.430 | 95.505 | 268.300 |
| | Sr[ppb] | 248 | 31.840 | 80.094 | 8.950 | 13.440 | 79.124 |
| | As[ppb] | 248 | 2.876 | 7.313 | 2.704 | 0.279 | 27.591 |
| | Mo[ppb] | 248 | 1.416 | 0.606 | 0.779 | 0.319 | 5.208 |
| | Cs[ppb] | 248 | 0.826 | 0.054 | 0.233 | 0.398 | 2.014 |
| | Co[ppb] | 248 | 0.201 | 0.081 | 0.284 | 0.025 | 3.504 |
| | Ag[ppb] | 248 | 0.440 | 0.189 | 0.434 | 0.025 | 2.745 |
| PANCREATIC CANCER | AGE | 155 | 59.994 | 103.201 | 10.159 | 34.000 | 84.000 |
| | Na[ppm] | 155 | 3243.595 | 9650.339 | 98.236 | 2963.369 | 3608.707 |
| | Mg[ppm] | 155 | 20.971 | 2.545 | 1.595 | 16.910 | 24.981 |
| | P[ppm] | 155 | 121.090 | 182.516 | 13.510 | 84.165 | 164.762 |
| | S[ppm] | 155 | 1025.570 | 5516.395 | 74.272 | 822.112 | 1233.784 |
| | K[ppm] | 155 | 158.398 | 173.821 | 13.184 | 131.812 | 217.662 |
| | Ca[ppm] | 155 | 94.115 | 32.205 | 5.675 | 79.789 | 120.000 |
| | Fe[ppb] | 155 | 926.482 | 184468.883 | 429.498 | 163.043 | 2735.406 |
| | Cu[ppb] | 155 | 1009.521 | 46342.026 | 215.272 | 228.880 | 1856.322 |
| | Zn[ppb] | 155 | 734.491 | 17893.503 | 133.767 | 347.846 | 1201.938 |
| | Se[ppb] | 155 | 139.125 | 362.584 | 19.042 | 89.107 | 204.675 |
| | Rb[ppb] | 155 | 143.625 | 710.703 | 26.659 | 87.956 | 268.869 |
| | Sr[ppb] | 155 | 31.935 | 94.243 | 9.708 | 14.586 | 64.445 |
| | As[ppb] | 155 | 3.065 | 6.274 | 2.505 | 0.437 | 18.155 |
| | Mo[ppb] | 155 | 1.909 | 0.638 | 0.799 | 0.389 | 5.588 |
| | Cs[ppb] | 155 | 0.661 | 0.042 | 0.204 | 0.291 | 1.618 |
| | Co[ppb] | 155 | 0.200 | 0.045 | 0.212 | 0.055 | 2.124 |
| | Ag[ppb] | 155 | 0.562 | 0.631 | 0.794 | 0.049 | 5.744 |

# FIG. 16 EXAMPLE 4 (ENDOMETRIAL CANCER, FEMALE)

## TABLE 33
VARIABLES CONTAINED IN DISCRIMINANT FUNCTION

| VARIABLE | Wilks' lambda | F VALUE | DOF1 | DOF2 | P VALUE | *: P<0.05<br>**: P<0.01 |
|---|---|---|---|---|---|---|
| AGE | 0.9074 | 39.2096 | 1 | 384 | p < 0.001 | ** |
| Na[ppm] | 0.9909 | 3.5326 | 1 | 384 | 0.0609 | |
| Mg[ppm] | 0.9642 | 14.2404 | 1 | 384 | p < 0.001 | ** |
| P[ppm] | 0.9992 | 0.3058 | 1 | 384 | 0.5806 | |
| S[ppm] | 0.8966 | 44.2953 | 1 | 384 | p < 0.001 | ** |
| K[ppm] | 0.9468 | 21.5820 | 1 | 384 | p < 0.001 | ** |
| Ca[ppm] | 0.9888 | 4.3467 | 1 | 384 | 0.0377 | * |
| Fe[ppb] | 0.9900 | 3.8771 | 1 | 384 | 0.0497 | * |
| Cu[ppb] | 0.9992 | 0.3102 | 1 | 384 | 0.5779 | |
| Zn[ppb] | 0.9993 | 0.2789 | 1 | 384 | 0.5977 | |
| Se[ppb] | 0.9984 | 0.6184 | 1 | 384 | 0.4321 | |
| Rb[ppb] | 0.9970 | 1.1555 | 1 | 384 | 0.2831 | |
| Sr[ppb] | 1.0000 | 0.0181 | 1 | 384 | 0.8930 | |
| As[ppb] | 1.0000 | 0.0000 | 1 | 384 | 0.9971 | |
| Mo[ppb] | 0.9563 | 17.5318 | 1 | 384 | p < 0.001 | ** |
| Cs[ppb] | 0.9954 | 1.7665 | 1 | 384 | 0.1846 | |
| Co[ppb] | 0.9918 | 3.1694 | 1 | 384 | 0.0758 | |
| Ag[ppb] | 0.9980 | 0.7513 | 1 | 384 | 0.3866 | |

・DOF: Degree of Freedom

## TABLE 34
DISCRIMINANT COEFFICIENTS

| VARIABLE | FUNCTION 1 |
|---|---|
| AGE | -0.0449 |
| Na[ppm] | 0.0012 |
| Mg[ppm] | 0.1728 |
| P[ppm] | 0.0030 |
| S[ppm] | 0.0081 |
| K[ppm] | 0.0296 |
| Ca[ppm] | -0.0369 |
| Fe[ppb] | -0.0004 |
| Cu[ppb] | -0.0002 |
| Zn[ppb] | 0.0003 |
| Se[ppb] | 0.0029 |
| Rb[ppb] | 0.0040 |
| Sr[ppb] | 0.0010 |
| As[ppb] | -0.0001 |
| Mo[ppb] | -0.3634 |
| Cs[ppb] | 0.5401 |
| Co[ppb] | -0.4894 |
| Ag[ppb] | -0.0997 |
| CONSTANT TERM | -16.5526 |

# FIG. 17 EXAMPLE 4 (ENDOMETRIAL CANCER, FEMALE)

TABLE 35

CENTROID OF EACH GROUP

| STATISTICAL DATA | FUNCTION 1 |
|---|---|
| CONTROL | 0.9216 |
| ENDOMETRIAL CANCER | −1.4745 |

TABLE 36

DISCRIMINATION RESULT

| | | PREDICTED VALUE | | PERCENTAGE OF CORRECT CLASSIFICA-TIONS |
|---|---|---|---|---|
| | | CONTROL | ENDOMETRIAL CANCER | |
| OBSERVED VALUE | CONTROL | 222 | 26 | 89.52% |
| | ENDOMETRIAL CANCER | 14 | 141 | 90.97% |
| | | | OVERALL | 90.07% |

# FIG. 18 EXAMPLE 5 (BREAST CANCER, FEMALE)

## ⑤BREAST CANCER AND CONTROL

TABLE 41

| STATISTICAL DATA | n | % |
|---|---|---|
| CONTROL | 248 | 61.23% |
| BREAST CANCER | 157 | 38.77% |
| TOTAL | 405 | 100.00% |

TABLE 42

FUNDAMENTAL STATISTICS

| OBJECTIVE VARIABLE | VARIABLE | n | MEAN | UNBIASED VARIANCE | STANDARD DEVIATION | MINIMUM | MAXIMUM |
|---|---|---|---|---|---|---|---|
| CONTROL | AGE | 248 | 52.702 | 130.728 | 11.434 | 30.000 | 70.000 |
| | Na[ppm] | 248 | 3345.250 | 21624.906 | 147.054 | 2931.025 | 3865.949 |
| | Mg[ppm] | 248 | 22.428 | 2.943 | 1.715 | 17.369 | 27.633 |
| | P[ppm] | 248 | 131.433 | 212.523 | 14.578 | 93.752 | 184.009 |
| | S[ppm] | 248 | 1136.034 | 4613.853 | 67.925 | 950.359 | 1414.263 |
| | K[ppm] | 248 | 174.254 | 218.855 | 14.794 | 134.569 | 235.392 |
| | Ca[ppm] | 248 | 98.745 | 29.529 | 5.434 | 78.869 | 124.393 |
| | Fe[ppb] | 248 | 913.462 | 123191.867 | 350.987 | 80.915 | 2468.603 |
| | Cu[ppb] | 248 | 1007.381 | 22727.952 | 150.758 | 635.642 | 1772.913 |
| | Zn[ppb] | 248 | 792.517 | 12898.719 | 113.573 | 496.980 | 1109.072 |
| | Se[ppb] | 248 | 149.481 | 477.199 | 21.845 | 111.674 | 282.918 |
| | Rb[ppb] | 248 | 175.792 | 866.111 | 29.430 | 95.505 | 268.300 |
| | Sr[ppb] | 248 | 31.840 | 80.094 | 8.950 | 13.440 | 79.124 |
| | As[ppb] | 248 | 2.876 | 7.313 | 2.704 | 0.279 | 27.591 |
| | Mo[ppb] | 248 | 1.416 | 0.606 | 0.779 | 0.319 | 5.208 |
| | Cs[ppb] | 248 | 0.826 | 0.054 | 0.233 | 0.398 | 2.014 |
| | Co[ppb] | 248 | 0.201 | 0.081 | 0.284 | 0.025 | 3.504 |
| | Ag[ppb] | 248 | 0.440 | 0.189 | 0.434 | 0.025 | 2.745 |
| PANCREATIC CANCER | AGE | 157 | 58.352 | 135.134 | 11.625 | 27.319 | 87.710 |
| | Na[ppm] | 157 | 3259.483 | 15406.846 | 124.124 | 2886.890 | 3511.222 |
| | Mg[ppm] | 157 | 21.303 | 2.238 | 1.496 | 17.734 | 25.664 |
| | P[ppm] | 157 | 122.489 | 155.494 | 12.470 | 94.727 | 160.053 |
| | S[ppm] | 157 | 1062.133 | 5358.477 | 73.202 | 859.977 | 1230.381 |
| | K[ppm] | 157 | 174.916 | 3323.744 | 57.652 | 139.628 | 671.430 |
| | Ca[ppm] | 157 | 95.269 | 27.923 | 5.284 | 83.123 | 119.810 |
| | Fe[ppb] | 157 | 1248.891 | 586358.258 | 765.740 | 158.521 | 6656.016 |
| | Cu[ppb] | 157 | 974.892 | 25945.975 | 161.078 | 630.391 | 2004.917 |
| | Zn[ppb] | 157 | 854.041 | 14347.897 | 119.783 | 559.811 | 1231.696 |
| | Se[ppb] | 157 | 147.925 | 633.060 | 25.161 | 111.158 | 364.813 |
| | Rb[ppb] | 157 | 158.716 | 952.202 | 30.858 | 94.532 | 278.563 |
| | Sr[ppb] | 157 | 31.499 | 98.087 | 9.904 | 15.389 | 77.340 |
| | As[ppb] | 157 | 3.233 | 3.313 | 1.820 | 0.643 | 13.813 |
| | Mo[ppb] | 157 | 1.241 | 0.144 | 0.380 | 0.488 | 2.605 |
| | Cs[ppb] | 157 | 0.684 | 0.042 | 0.205 | 0.260 | 1.772 |
| | Co[ppb] | 157 | 0.151 | 0.015 | 0.124 | 0.000 | 0.876 |
| | Ag[ppb] | 157 | 0.512 | 0.320 | 0.565 | 0.045 | 3.621 |

# FIG. 19 EXAMPLE 5 (BREAST CANCER, FEMALE)

TABLE 43

VARIABLES CONTAINED IN DISCRIMINANT FUNCTION

| VARIABLE | Wilks' lambda | F VALUE | DOF1 | DOF2 | P VALUE | *: P<0.05<br>**: P<0.01 |
|---|---|---|---|---|---|---|
| AGE | 0.9084 | 38.9388 | 1 | 386 | p < 0.001 | ** |
| Na[ppm] | 0.9963 | 1.4379 | 1 | 386 | 0.2312 | |
| Mg[ppm] | 0.9620 | 15.2542 | 1 | 386 | p < 0.001 | ** |
| P[ppm] | 0.9891 | 4.2649 | 1 | 386 | 0.0396 | * |
| S[ppm] | 0.9365 | 26.1947 | 1 | 386 | p < 0.001 | ** |
| K[ppm] | 0.9995 | 0.1937 | 1 | 386 | 0.6601 | |
| Ca[ppm] | 0.9938 | 2.4216 | 1 | 386 | 0.1205 | |
| Fe[ppb] | 0.9484 | 21.0118 | 1 | 386 | p < 0.001 | ** |
| Cu[ppb] | 0.9908 | 3.5994 | 1 | 386 | 0.0585 | |
| Zn[ppb] | 0.9289 | 29.5356 | 1 | 386 | p < 0.001 | ** |
| Se[ppb] | 1.0000 | 0.0097 | 1 | 386 | 0.9217 | |
| Rb[ppb] | 0.9997 | 0.1207 | 1 | 386 | 0.7284 | |
| Sr[ppb] | 0.9993 | 0.2558 | 1 | 386 | 0.6133 | |
| As[ppb] | 0.9995 | 0.1755 | 1 | 386 | 0.6755 | |
| Mo[ppb] | 0.9941 | 2.2731 | 1 | 386 | 0.1325 | |
| Cs[ppb] | 0.9714 | 11.3520 | 1 | 386 | p < 0.001 | ** |
| Co[ppb] | 0.9988 | 0.4662 | 1 | 386 | 0.4951 | |
| Ag[ppb] | 0.9985 | 0.5817 | 1 | 386 | 0.4461 | |

・DOF: Degree of Freedom

TABLE 44

DISCRIMINANT COEFFICIENTS

| VARIABLE | FUNCTION 1 |
|---|---|
| AGE | -0.0478 |
| Na[ppm] | 0.0009 |
| Mg[ppm] | 0.2012 |
| P[ppm] | 0.0124 |
| S[ppm] | 0.0069 |
| K[ppm] | -0.0010 |
| Ca[ppm] | 0.0259 |
| Fe[ppb] | -0.0007 |
| Cu[ppb] | 0.0009 |
| Zn[ppb] | -0.0037 |
| Se[ppb] | 0.0003 |
| Rb[ppb] | 0.0012 |
| Sr[ppb] | 0.0041 |
| As[ppb] | -0.0135 |
| Mo[ppb] | 0.1726 |
| Cs[ppb] | 1.3479 |
| Co[ppb] | 0.2230 |
| Ag[ppb] | -0.1165 |
| CONSTANT TERM | -14.9771 |

# FIG. 20 EXAMPLE 5 (BREAST CANCER, FEMALE)

TABLE 45

CENTROID OF EACH GROUP

| STATISTICAL DATA | FUNCTION 1 |
|---|---|
| CONTROL | 0.7859 |
| BREAST CANCER | -1.2414 |

TABLE 46

DISCRIMINATION RESULT

| | | PREDICTED VALUE | | PERCENTAGE OF CORRECT CLASSIFICATIONS |
|---|---|---|---|---|
| | | CONTROL | BREAST CANCER | |
| OBSERVED VALUE | CONTROL | 207 | 41 | 83.47% |
| | BREAST CANCER | 21 | 136 | 86.62% |
| | | | OVERALL | 84.69% |

# FIG. 21 EXAMPLE 6 (COLORECTAL CANCER, FEMALE)

## ⑥COLORECTAL CANCER AND CONTROL

TABLE 51

| STATISTICAL DATA | n | % |
|---|---|---|
| CONTROL | 248 | 62.31% |
| COLORECTAL CANCER | 150 | 37.69% |
| TOTAL | 398 | 100.00% |

TABLE 52

FUNDAMENTAL STATISTICS

| OBJECTIVE VARIABLE | VARIABLE | n | MEAN | UNBIASED VARIANCE | STANDARD DEVIATION | MINIMUM | MAXIMUM |
|---|---|---|---|---|---|---|---|
| CONTROL | AGE | 248 | 52.702 | 130.728 | 11.434 | 30.000 | 70.000 |
| | Na[ppm] | 248 | 3345.250 | 21624.906 | 147.054 | 2931.025 | 3865.949 |
| | Mg[ppm] | 248 | 22.428 | 2.943 | 1.715 | 17.369 | 27.633 |
| | P[ppm] | 248 | 131.433 | 212.523 | 14.578 | 93.752 | 184.009 |
| | S[ppm] | 248 | 1136.034 | 4613.853 | 67.925 | 950.359 | 1414.263 |
| | K[ppm] | 248 | 174.254 | 218.855 | 14.794 | 134.569 | 235.392 |
| | Ca[ppm] | 248 | 98.745 | 29.529 | 5.434 | 78.869 | 124.393 |
| | Fe[ppb] | 248 | 913.462 | 123191.867 | 350.987 | 80.915 | 2468.603 |
| | Cu[ppb] | 248 | 1007.381 | 22727.952 | 150.758 | 635.642 | 1772.913 |
| | Zn[ppb] | 248 | 792.517 | 12898.719 | 113.573 | 496.980 | 1109.072 |
| | Se[ppb] | 248 | 149.481 | 477.199 | 21.845 | 111.674 | 282.918 |
| | Rb[ppb] | 248 | 175.792 | 866.111 | 29.430 | 95.505 | 268.300 |
| | Sr[ppb] | 248 | 31.840 | 80.094 | 8.950 | 13.440 | 79.124 |
| | As[ppb] | 248 | 2.876 | 7.313 | 2.704 | 0.279 | 27.591 |
| | Mo[ppb] | 248 | 1.416 | 0.606 | 0.779 | 0.319 | 5.208 |
| | Cs[ppb] | 248 | 0.826 | 0.054 | 0.233 | 0.398 | 2.014 |
| | Co[ppb] | 248 | 0.201 | 0.081 | 0.284 | 0.025 | 3.504 |
| | Ag[ppb] | 248 | 0.440 | 0.189 | 0.434 | 0.025 | 2.745 |
| PANCREATIC CANCER | AGE | 150 | 64.062 | 91.906 | 9.587 | 32.304 | 84.134 |
| | Na[ppm] | 150 | 3421.386 | 17443.712 | 132.075 | 3139.210 | 4008.909 |
| | Mg[ppm] | 150 | 22.782 | 5.380 | 2.319 | 14.698 | 29.098 |
| | P[ppm] | 150 | 123.527 | 223.340 | 14.945 | 79.558 | 171.941 |
| | S[ppm] | 150 | 1069.825 | 9021.091 | 94.979 | 815.302 | 1580.363 |
| | K[ppm] | 150 | 174.074 | 251.421 | 15.856 | 125.838 | 230.514 |
| | Ca[ppm] | 150 | 97.151 | 31.239 | 5.589 | 85.743 | 128.195 |
| | Fe[ppb] | 150 | 949.840 | 217978.072 | 466.881 | 125.507 | 2846.631 |
| | Cu[ppb] | 150 | 1106.018 | 56597.363 | 237.902 | 496.083 | 2107.212 |
| | Zn[ppb] | 150 | 818.594 | 16665.855 | 129.096 | 502.776 | 1366.125 |
| | Se[ppb] | 150 | 148.325 | 531.994 | 23.065 | 59.184 | 261.804 |
| | Rb[ppb] | 150 | 154.315 | 844.454 | 29.059 | 55.925 | 215.412 |
| | Sr[ppb] | 150 | 32.652 | 128.928 | 11.355 | 15.529 | 86.415 |
| | As[ppb] | 150 | 3.929 | 13.207 | 3.634 | 0.198 | 32.780 |
| | Mo[ppb] | 150 | 1.329 | 0.246 | 0.496 | 0.306 | 3.168 |
| | Cs[ppb] | 150 | 0.685 | 0.039 | 0.197 | 0.175 | 1.265 |
| | Co[ppb] | 150 | 0.233 | 0.095 | 0.308 | 0.056 | 3.237 |
| | Ag[ppb] | 150 | 0.652 | 0.713 | 0.844 | 0.069 | 5.805 |

# FIG. 22 EXAMPLE 6 (COLORECTAL CANCER, FEMALE)

## TABLE 53
## VARIABLES CONTAINED IN DISCRIMINANT FUNCTION

| VARIABLE | Wilks' lambda | F VALUE | DOF1 | DOF2 | P VALUE | *: P<0.05<br>**: P<0.01 |
|---|---|---|---|---|---|---|
| AGE | 0.9404 | 24.0141 | 1 | 379 | p < 0.001 | ** |
| Na[ppm] | 0.8904 | 46.6485 | 1 | 379 | p < 0.001 | ** |
| Mg[ppm] | 0.9927 | 2.7907 | 1 | 379 | 0.0956 | |
| P[ppm] | 0.9878 | 4.6959 | 1 | 379 | 0.0309 | * |
| S[ppm] | 0.8625 | 60.4283 | 1 | 379 | p < 0.001 | ** |
| K[ppm] | 0.9921 | 3.0238 | 1 | 379 | 0.0829 | |
| Ca[ppm] | 0.9898 | 3.9196 | 1 | 379 | 0.0484 | * |
| Fe[ppb] | 0.9887 | 4.3462 | 1 | 379 | 0.0378 | * |
| Cu[ppb] | 0.9754 | 9.5387 | 1 | 379 | 0.0022 | ** |
| Zn[ppb] | 0.9448 | 22.1587 | 1 | 379 | p < 0.001 | ** |
| Se[ppb] | 0.9946 | 2.0755 | 1 | 379 | 0.1505 | |
| Rb[ppb] | 0.9981 | 0.7380 | 1 | 379 | 0.3908 | |
| Sr[ppb] | 0.9981 | 0.7159 | 1 | 379 | 0.3980 | |
| As[ppb] | 0.9846 | 5.9259 | 1 | 379 | 0.0154 | * |
| Mo[ppb] | 0.9965 | 1.3289 | 1 | 379 | 0.2497 | |
| Cs[ppb] | 0.9770 | 8.9265 | 1 | 379 | 0.0030 | ** |
| Co[ppb] | 0.9979 | 0.7816 | 1 | 379 | 0.3772 | |
| Ag[ppb] | 0.9809 | 7.3706 | 1 | 379 | 0.0069 | ** |

・DOF: Degree of Freedom

## TABLE 54
## DISCRIMINANT COEFFICIENTS

| VARIABLE | FUNCTION 1 |
|---|---|
| AGE | 0.0374 |
| Na[ppm] | 0.0044 |
| Mg[ppm] | −0.0731 |
| P[ppm] | −0.0124 |
| S[ppm] | −0.0089 |
| K[ppm] | 0.0114 |
| Ca[ppm] | −0.0384 |
| Fe[ppb] | 0.0004 |
| Cu[ppb] | 0.0013 |
| Zn[ppb] | 0.0033 |
| Se[ppb] | 0.0055 |
| Rb[ppb] | −0.0032 |
| Sr[ppb] | −0.0064 |
| As[ppb] | 0.0589 |
| Mo[ppb] | −0.1266 |
| Cs[ppb] | −1.2615 |
| Co[ppb] | 0.2309 |
| Ag[ppb] | 0.3201 |
| CONSTANT TERM | −5.7210 |

# FIG. 23 EXAMPLE 6 (COLORECTAL CANCER, FEMALE)

TABLE 55

CENTROID OF EACH GROUP

| STATISTICAL DATA | FUNCTION 1 |
|---|---|
| CONTROL | -0.7885 |
| COLORECTAL CANCER | 1.3037 |

TABLE 56

DISCRIMINATION RESULT

| | | PREDICTED VALUE | | PERCENTAGE OF CORRECT CLASSIFICA-TIONS |
|---|---|---|---|---|
| | | CONTROL | COLORECTAL CANCER | |
| OBSERVED VALUE | CONTROL | 212 | 36 | 85.48% |
| | COLORECTAL CANCER | 21 | 129 | 86.00% |
| | | | OVERALL | 85.68% |

# FIG. 24 EXAMPLES 1-3

ROC CURVES OF PANCREATIC CANCER, COLORECTAL CANCER, AND PROSTATE CANCER (MALE)

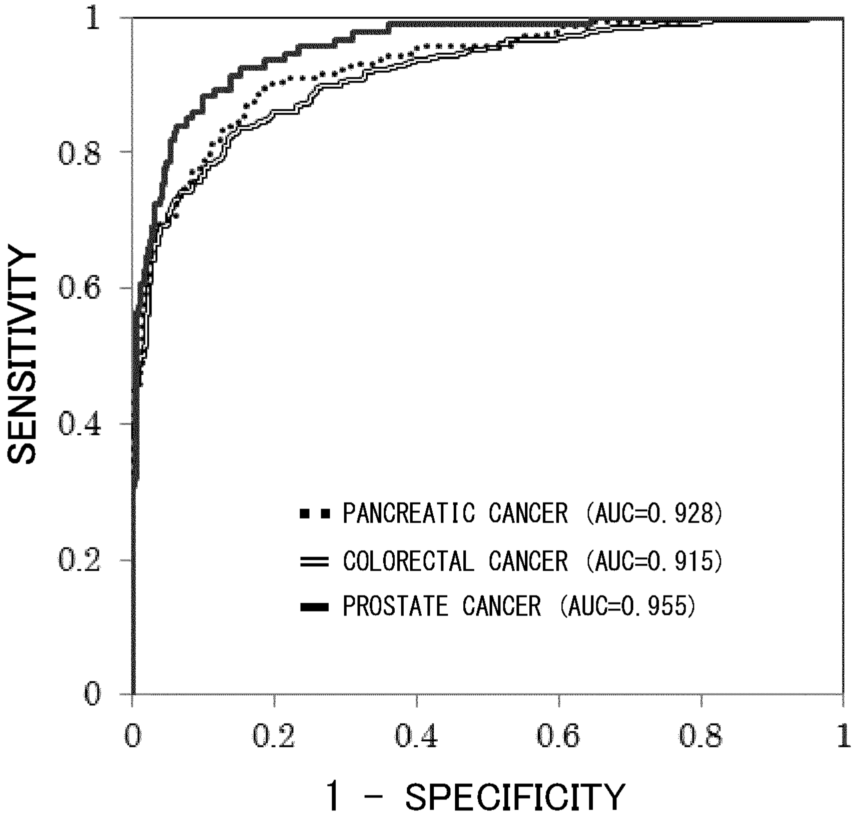

FIG. 25 EXAMPLES 4-6

ROC CURVES OF BREAST CANCER, ENDOMETRIAL CANCER, AND COLORECTAL CANCER (FEMALE)

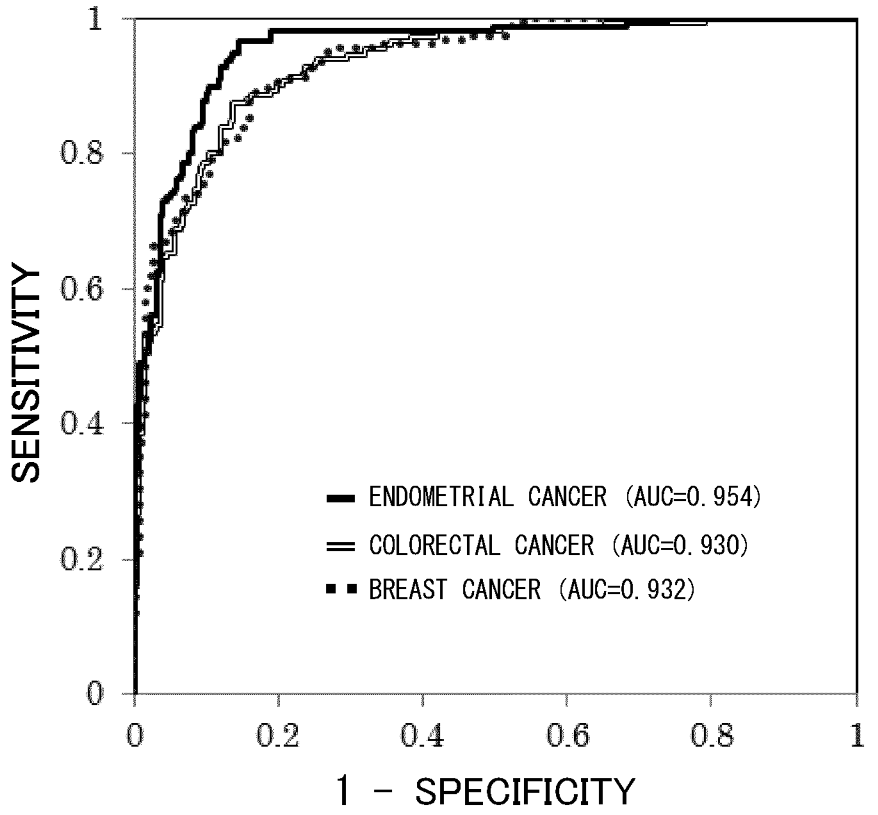

FIG. 26 EXAMPLE 1 (PANCREATIC CANCER, MALE)

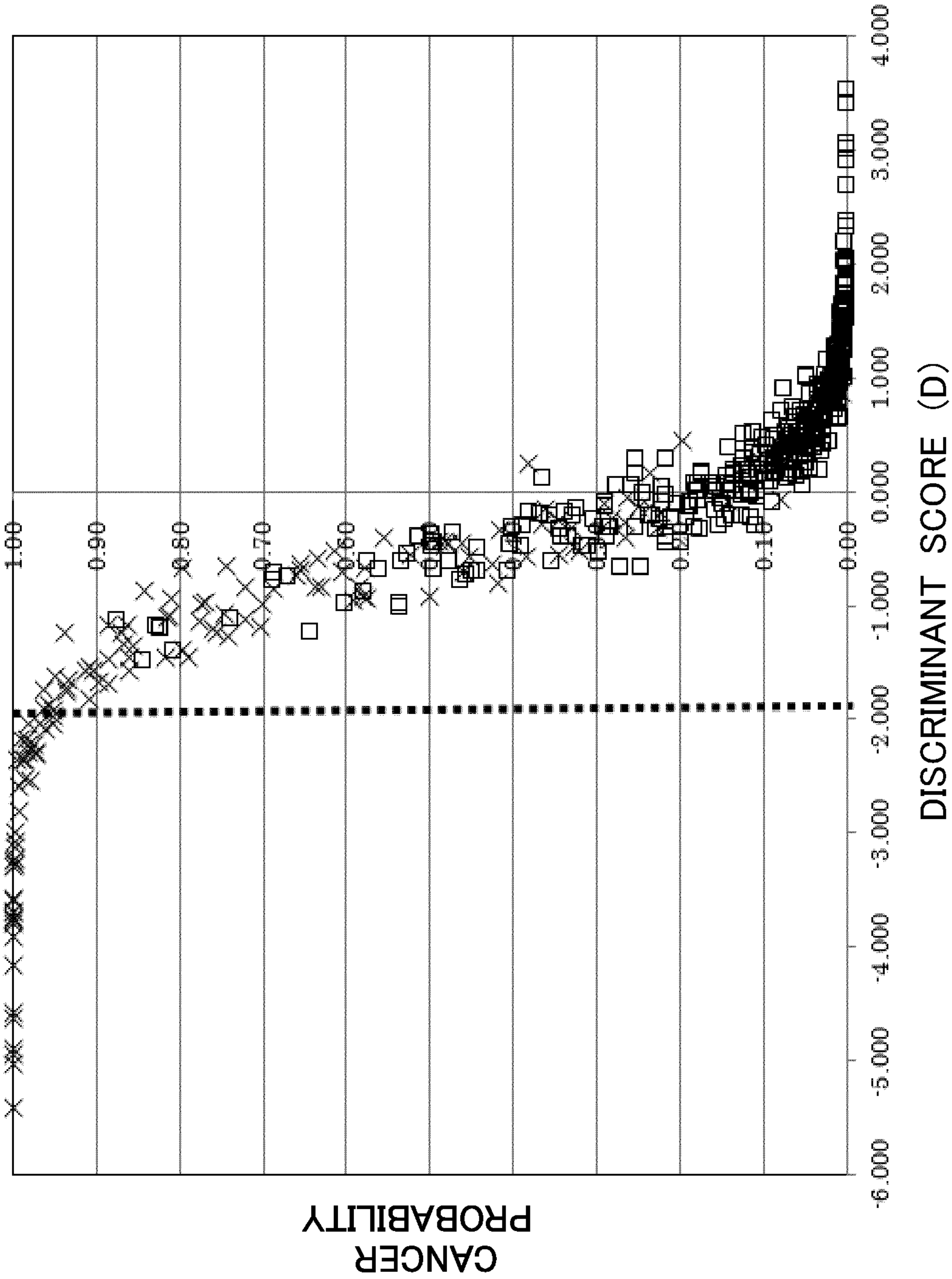

FIG. 27 EXAMPLE 2 (PROSTATE CANCER, MALE)

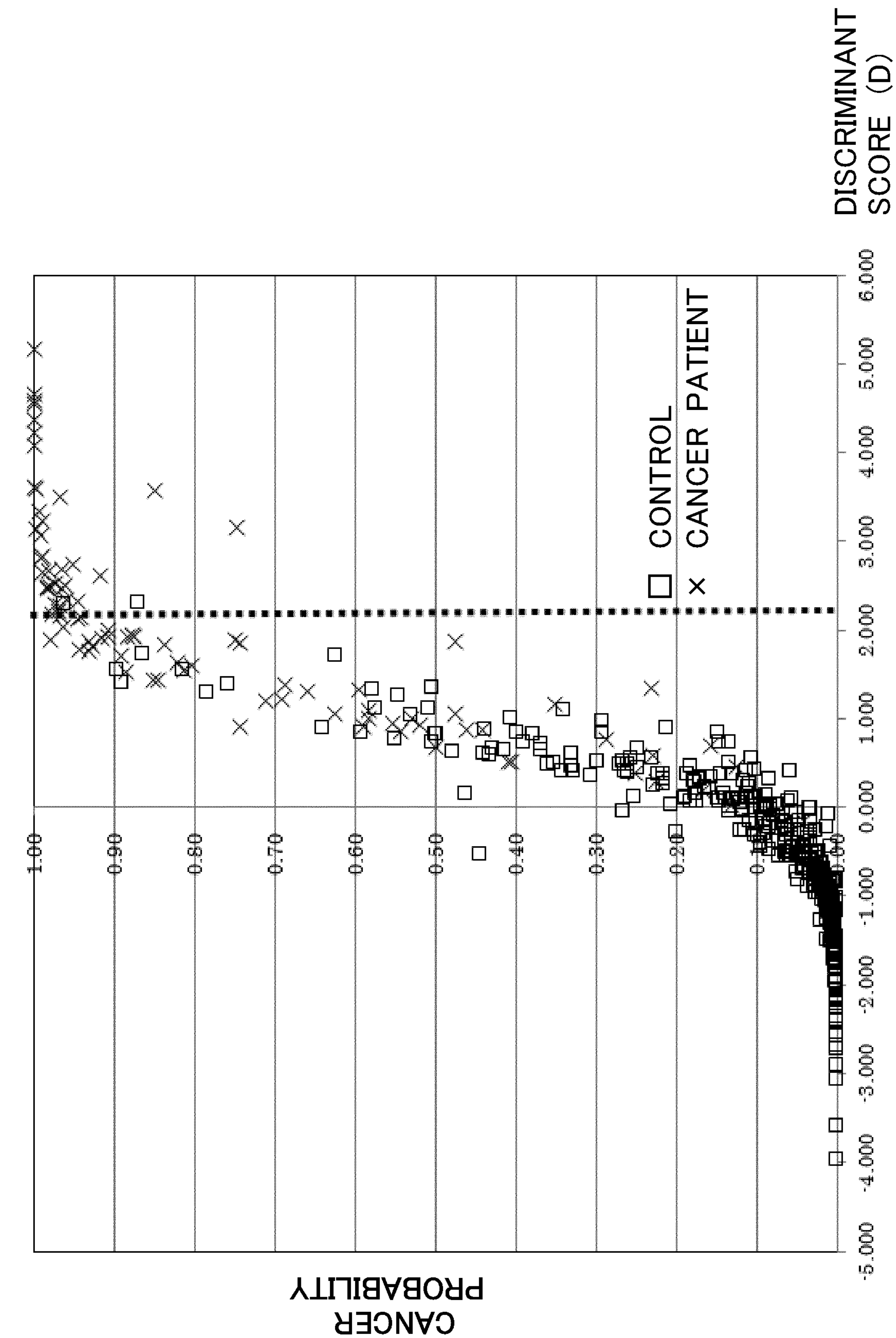

FIG. 28 EXAMPLE 3 (COLORECTAL CANCER, MALE)

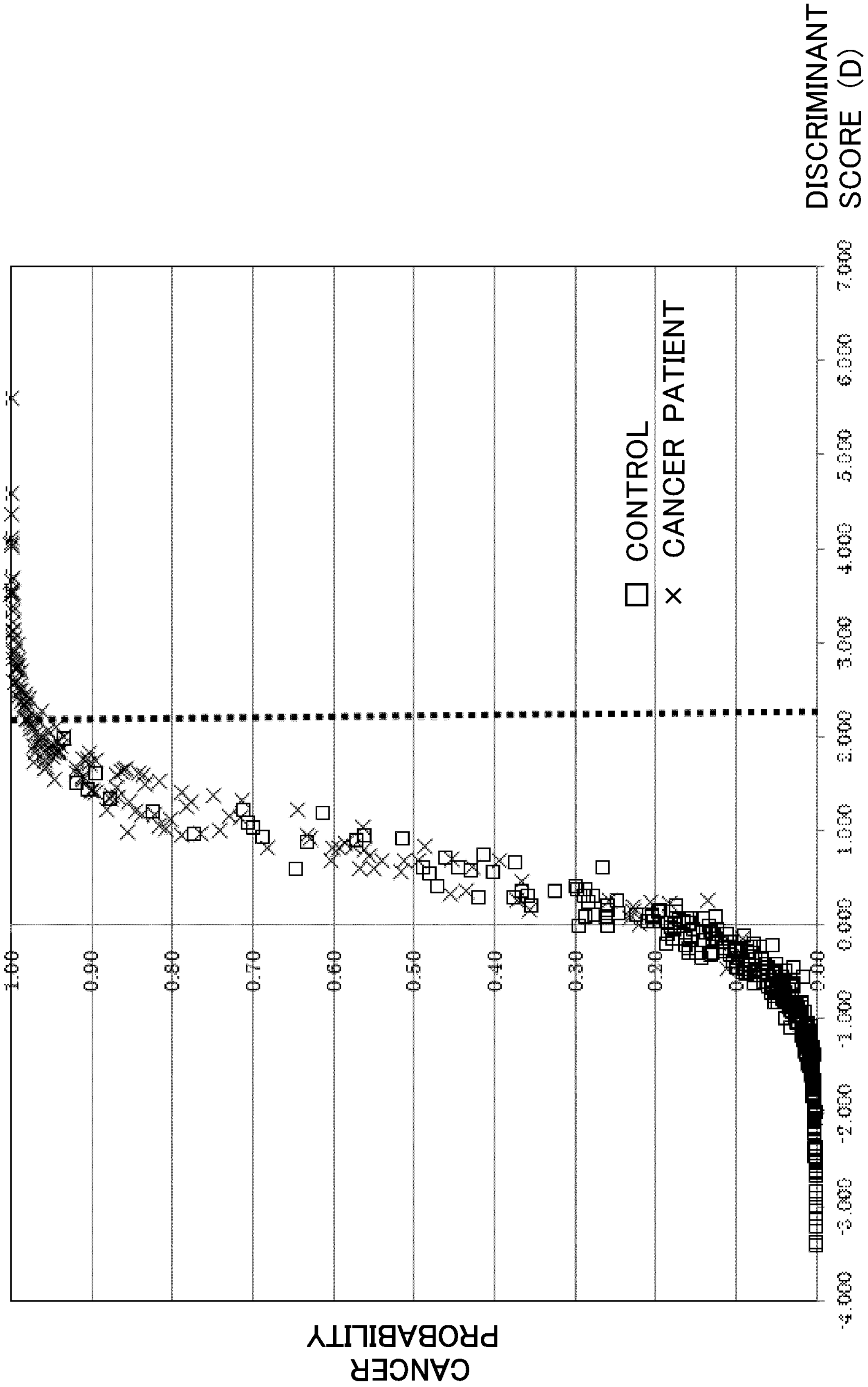

FIG. 29 EXAMPLE 4 (ENDMETRIAL CANCER, FEMALE)

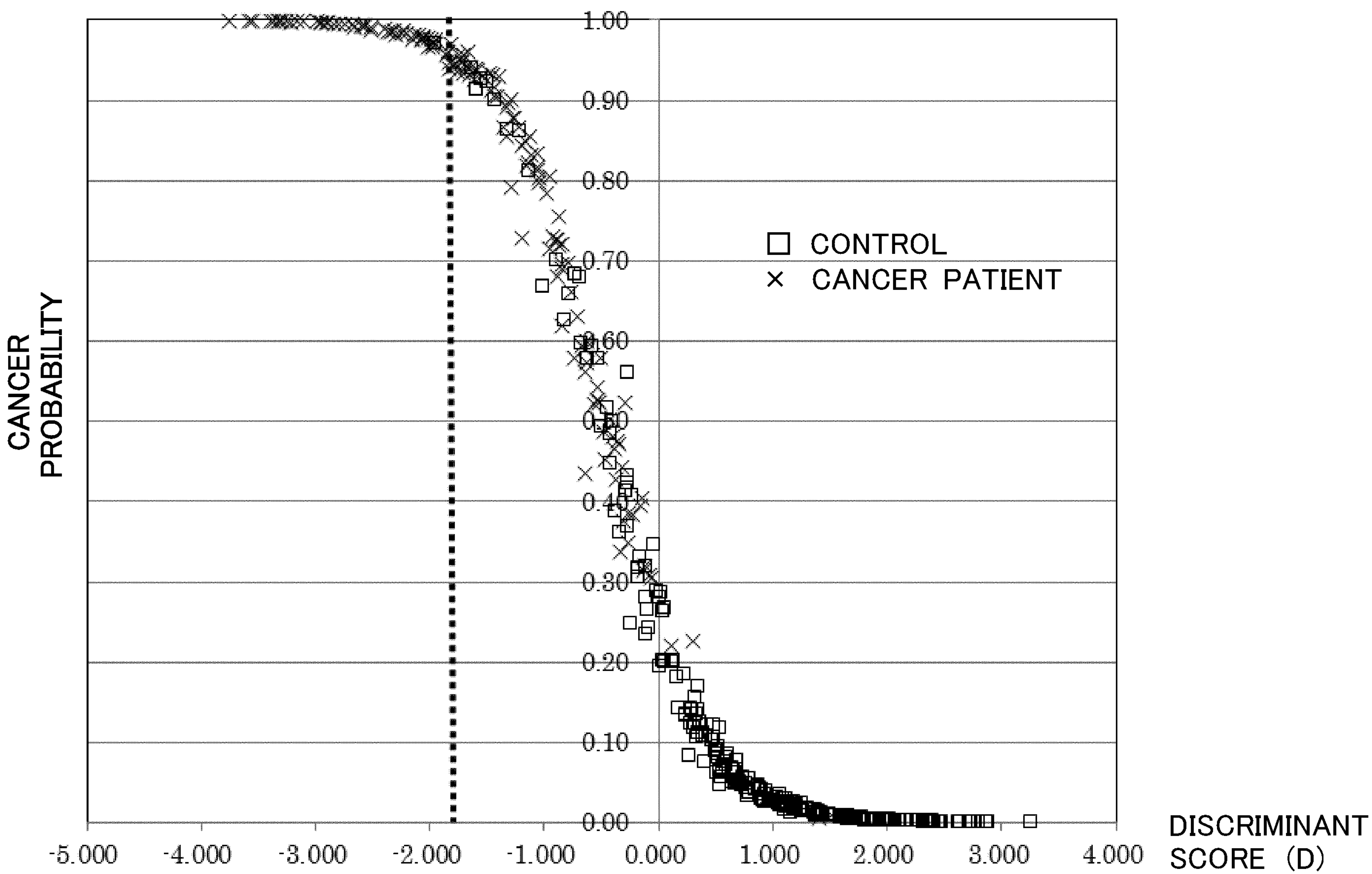

FIG. 30 EXAMPLE 5 (BREAST CANCER, FEMALE)

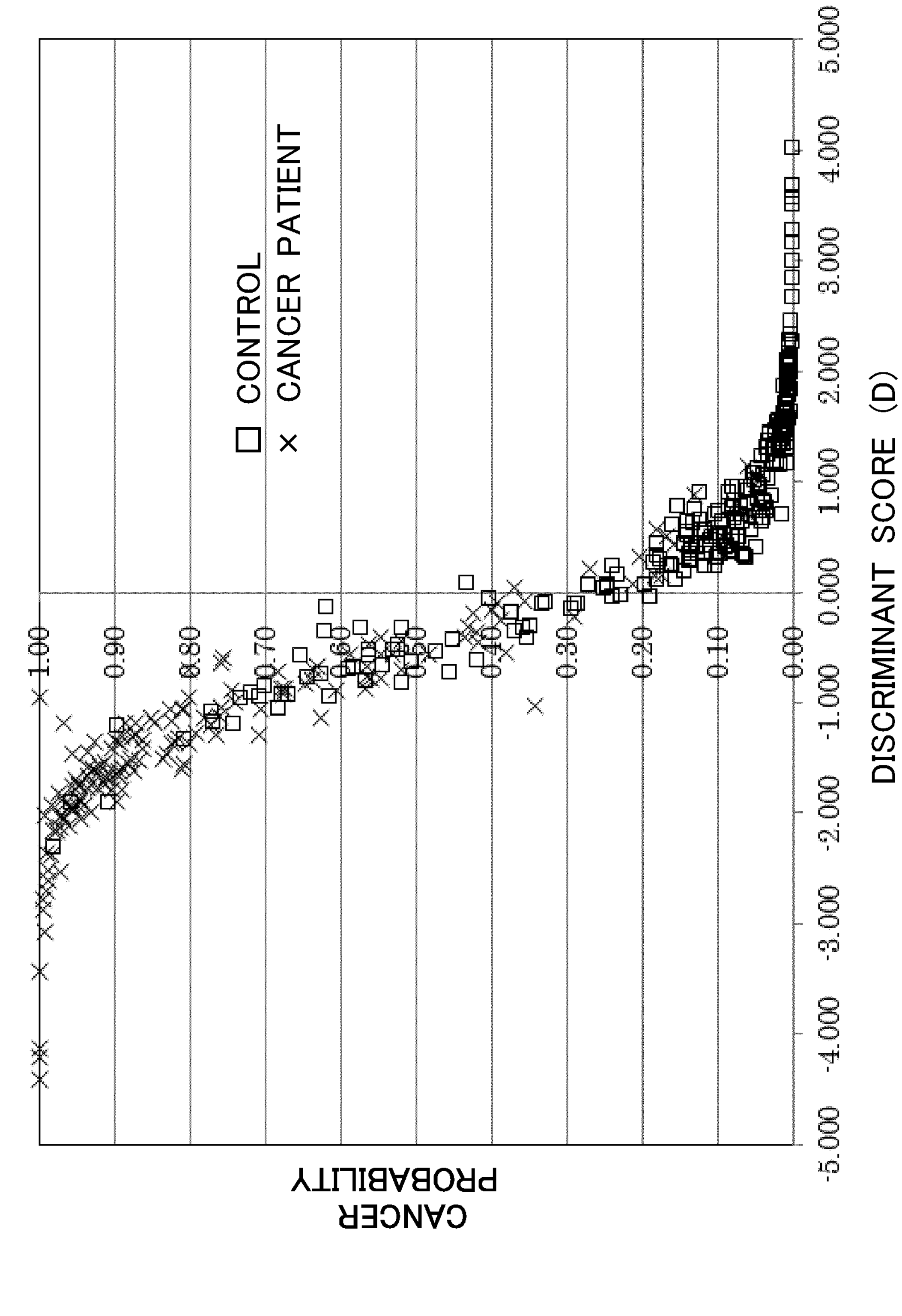

FIG. 31 EXAMPLE 6 (COLORECTAL CANCER, FEMALE)

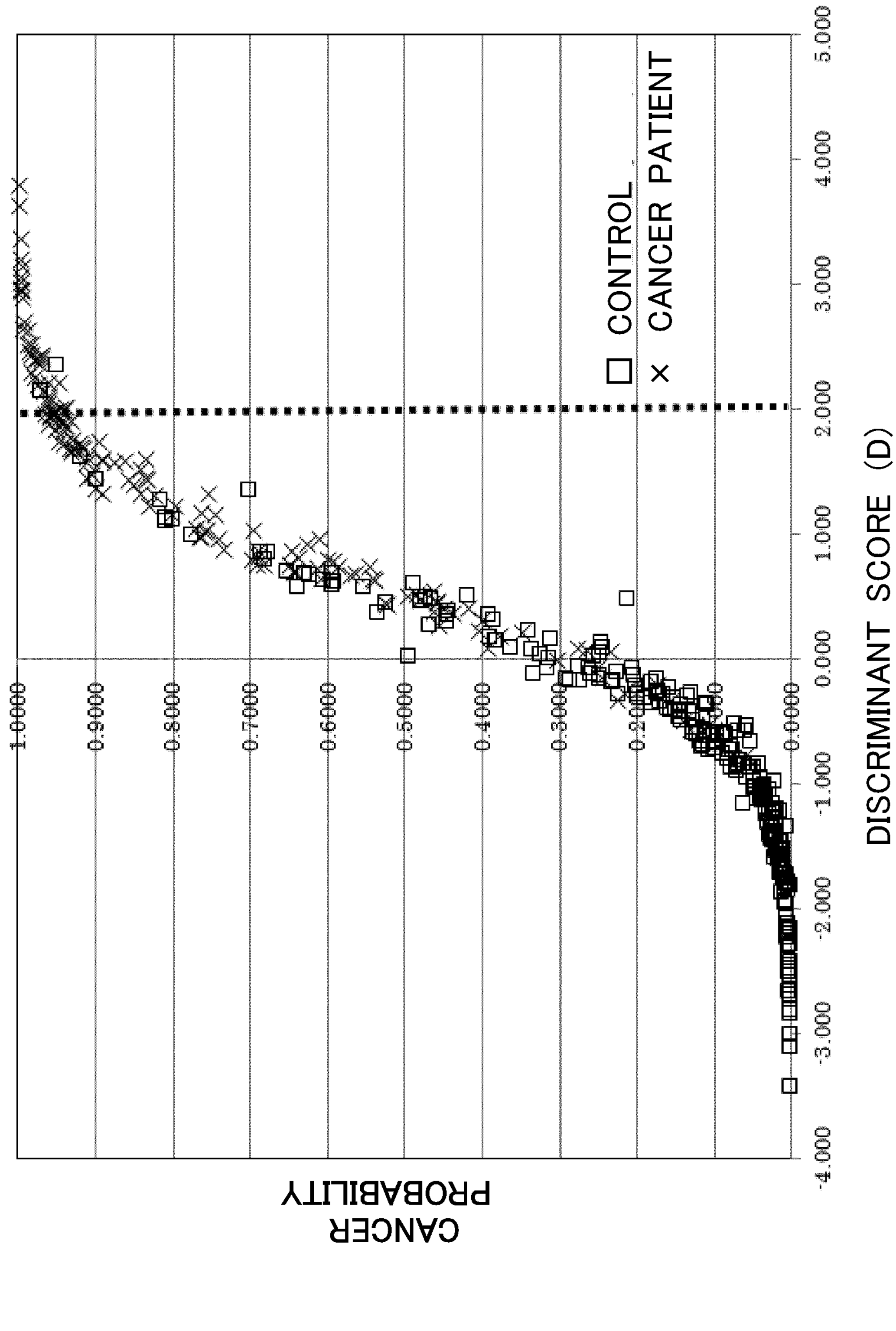

## INTERNATIONAL SEARCH REPORT

International application No.
PCT/JP2018/035978

A. CLASSIFICATION OF SUBJECT MATTER
Int.Cl. G01N33/49(2006.01)i

According to International Patent Classification (IPC) or to both national classification and IPC

B. FIELDS SEARCHED

Minimum documentation searched (classification system followed by classification symbols)
Int.Cl. G01N33/49

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X/A | WO 2016/042805 A1 (RENATECH CO., LTD.) 24 March 2016, entire text, all drawings, in particular, see claims, paragraphs [0070]-[0080], fig. 1, 14, etc. & US 2017/0254821 A1, entire text, all drawings, in particular, see claims, paragraphs [0111]-[0121], fig. 1, 14, etc. & EP 3182132 A1 | 1, 8/2-7, 9-14 |
| A | JP 2011-047715 A (LA BELLE VIE KK) 10 March 2011, entire text, all drawings, in particular, see claims, etc. (Family: none) | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 January 2019 (09.01.2019) | 22 January 2019 (22.01.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/035978

C (Continuation). DOCUMENTS CONSIDERED TO BE RELEVANT

| Category* | Citation of document, with indication, where appropriate, of the relevant passages | Relevant to claim No. |
|---|---|---|
| A | JP 2013-221866 A (HYOGO SCIENCE AND TECHNOLOGY ASSOCIATION) 28 October 2013, entire text, all drawings, in particular, see claims, paragraphs [0005]-[0009], etc. (Family: none) | 1-14 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5470848 B
- JP 6082478 B


### Non-patent literature cited in the description


- **GUPTA SK et al.** Serum trace elements and Cu/Zn ratio in breast cancer patients. *Journal of Surgical Oncology,* March 1991, vol. 46 (3), 178-181
- **NECIP PIRINCCI et al.** Levels of Serum Trace Elements in Renal Cell Carcinoma Cases. *Asian Pacific Journal of Cancer Prevention,* 2013, vol. 14 (1), 499-502